# EUROPEAN PATENT APPLICATION

(11) **EP 1 640 713 A1**
(43) Date of publication of application: **29.03.2006**
(21) Application number: 05021125.9
(22) Date of filing: 28.09.2005
(51) Int. Cl.: G01N 27/407, G01N 27/04, G01N 27/406

(54) **Gas sensor and gas concentration measurement methods using a said gas sensor**

(30) Priority: 28.09.2004 JP 2004282450; 28.09.2004 JP 2004282459
(71) Applicant: TDK Corporation, Tokyo 103-8272 (JP)
(72) Inventor: Itoh, Yuugi, Tokyo 103-8272 (JP)
(74) Representative: Grünecker, Kinkeldey, Stockmair & Schwanhäusser Anwaltssozietät

(57) **Abstract**

A gas sensor comprising: a solid electrolyte member (3); a sensing electrode (4) provided on the solid electrolyte member (3) and comprising at least one of a metal carbonate and a metal hydrogen carbonate; and a plurality of reference electrodes (5a,5b) arranged on the solid electrolyte member (3).

## Description

### BACKGROUND OF THE INVENTION

The present invention relates to a gas concentration measurement method and a gas sensor.

### Field of the Invention

There are conventionally known electrochemical gas sensors. The gas sensors of this type use a sensor element in which a sensing electrode and a reference electrode are formed on a substrate-like solid electrolyte member. The sensing electrode has a metal layer as a current collector, and a metal carbonate layer or a metal hydrogen carbonate layer as a sensing material.

### Related Background of the Invention

Such gas sensors are able to measure a concentration of a measuring-object gas in the following manner. First, the measuring-object gas is adsorbed on the metal carbonate layer or the metal hydrogen carbonate layer in accordance with the concentration of the measuring-object gas. Next, an equilibrium reaction to generate ions originating from the measuring-object gas takes place in the metal carbonate layer or the metal hydrogen carbonate layer. A concentration difference of conductive ions occurs between the sensing electrode and the reference electrode in the solid electrolyte member and the concentration of the measuring-object gas can be measured based on an electromotive force (potential difference) caused by the concentration difference. At this time, the solid electrolyte member is used as heated at a temperature suitable for ion conduction (e.g., 350°C or higher) (cf Japanese Patent Application Laid-Open No. 11-295265). The electromotive force in the solid electrolyte member differs depending upon temperatures even at the same gas concentration. Therefore, the concentration value of the measuring-object gas based on the electromotive force is corrected according to the temperature of the solid electrolyte member.

### SUMMARY OF THE INVENTION

Incidentally, in the case of the common electrochemical gas sensors, where the reference electrode and the sensing electrode are arranged to have a large impedance between the reference electrode and the sensing electrode, the electromotive force value becomes large enough to accurately measure even a low concentration of the measuring-object gas. Where the reference electrode and sensing electrode are arranged to have a small impedance between the reference electrode and the sensing electrode, the reaction rate can be increased. In this manner, the preferred magnitude of the impedance between the reference electrode and the sensing electrode differs depending upon applications and operation environments of the gas sensors. However, the impedance between the reference electrode and the sensing electrode was constant in the conventional gas sensors, and there were no gas sensors capable of changing the impedance between the reference electrode and the sensing electrode according to applications. That is, the conventional gas sensors can not measure a gas concentration in various methods.

The conventional gas sensors use a temperature detecting element, for example, such as a thermistor or a thermocouple, in order to measure the temperature of the solid electrolyte member. In the case where the thermistor is used, the thermistor is located near the sensor element and measures the ambient temperature around the sensor element. In the case where the thermocouple is used, the thermocouple is kept in contact with the sensor element and measures the temperature of the sensor element itself.

However, where the ambient temperature is measured around the sensor element, it is different from the temperature of the solid electrolyte member and thus gives rise to a large measurement error. Where the thermocouple is kept in contact with the sensor element, heat leaks through the thermocouple from the sensor element, and it becomes difficult to keep the solid electrolyte member at the predetermined temperature. In addition, since the solid electrolyte member is used as heated at the high temperature of not less than 350°C as described above, it is also difficult to fix the thermocouple to the sensor element. Against such background, a gas sensor which can accurately measure a gas concentration according to the temperature without a temperature detecting element is demanded.

An object of the present invention is to provide a gas sensor capable of measuring a gas concentration in various methods, and to provide a gas concentration measurement method using the gas sensor.

One aspect of the present invention relates to a gas sensor. A first gas sensor according to the present invention is a gas sensor comprising: a solid electrolyte member; a sensing electrode provided on the solid electrolyte member and comprising at least one of a metal carbonate and a metal hydrogen carbonate; and first and second reference electrodes arranged on the solid electrolyte member.

According to the first gas sensor, the first and second reference electrodes are short-circuited by an internal wire or an external wire to enlarge the contact area between the reference electrode and the solid electrolyte member, whereby the impedance between the reference electrode and the sensing electrode becomes smaller than in the case where the first or second reference electrode is singly used. Therefore, this gas sensor is able to change the contact area between the reference electrode and the solid electrolyte member (i.e., the impedance between the reference electrode and the sensing electrode) in accordance with a measurement application or an operation environment. Hence, the first gas sensor can measure a gas concentration in various methods.

Incidentally, the impedance between a sensing electrode and a reference electrode provided on the solid electrolyte member depends upon the temperature of the solid electrolyte member. Accordingly, the electromotive force generated between the sensing electrode and the reference electrode also depends upon the temperature of the solid electrolyte member. The Inventors discovers that, in accordance with the impedance between a sensing electrode and a reference electrode provided on the solid electrolyte member, the degree of impedance change (change rates) relative to temperature change varies. Since the first gas sensor is able to change the impedance between the sensing electrode and the reference electrode, the temperature of the solid electrolyte member can be measured without use of the temperature detecting element on the basis of the difference of the electromotive forces at the reference electrode. Since the temperature of the solid electrolyte member can be directly measured, the temperature of the solid electrolyte member can be measured with higher accuracy than with use of the temperature detecting element. This permits the concentration of the measuring-object gas to be obtained easily and accurately.

The first gas sensor preferably further comprises switch means for short-circuiting the first reference electrode and the second reference electrode with each other.

The first gas sensor may be configured in a configuration further comprising amplifying means for amplifying a voltage between two input terminals, wherein one of the two input terminals of the amplifying means is electrically connected to the first reference electrode or the second reference electrode, and wherein the other of the two input terminals of the amplifying means is electrically connected to the sensing electrode. This permits the sensor to measure with higher accuracy, the first electromotive force between the first or the second reference electrode and the sensing electrode, or the second electromotive force between the first and second reference electrodes in the short-circuited state and the sensing electrode.

The first gas sensor preferably further comprises gas concentration measuring means for measuring a first electromotive force between one of the first and second reference electrodes and the sensing electrode, or a second electromotive force between the first and second reference electrodes in a mutually short-circuited state of the first and second reference electrodes, and the sensing electrode, and for obtaining a concentration of a measuring-object gas, based on a correlation between the first or second electromotive force and the concentration of the measuring-object gas.

The first gas sensor preferably further comprises gas concentration calculating means for measuring a first electromotive force between one of the first and second reference electrodes and the sensing electrode, and a second electromotive force between the first and second reference electrodes in a mutually short-circuited state of the first and second reference electrodes, and the sensing electrode, for obtaining a temperature of the solid electrolyte member on the basis of a difference between the first electromotive force and the second electromotive force, and for obtaining a concentration of a measuring-object gas, based on a correlation between the first or second electromotive force and the concentration of the measuring-object gas, corresponding to the temperature.

The first gas sensor may further comprise a heater for heating the solid electrolyte member; and temperature controlling means for controlling a supplied power to the heater.

The temperature controlling means may control the supplied power so that a temperature of the solid electrolyte member nears a preset temperature. In the gas sensor, with increase in the temperature of the solid electrolyte member, the reaction is promoted between the metal carbonate or the metal hydrogen carbonate of the sensing electrode and the measuring-object gas. With decrease in the temperature of the solid electrolyte member, the amount of heat to the solid electrolyte member can be decreased, so as to reduce the power consumption. This gas sensor is able to change the activeness of reaction and the power consumption according to a measurement application or an operation environment, by selecting the preset temperature of the solid electrolyte member.

The temperature controlling means may measure a first electromotive force between one of the first and second reference electrodes and the sensing electrode, and a second electromotive force between the first and second reference electrodes in a mutually short-circuited state of the first and second reference electrodes, and the sensing electrode, obtain a temperature of the solid electrolyte member on the basis of a difference between the first electromotive force and the second electromotive force, and control a supplied power to the heater so that the temperature nears a predetermined temperature.

A second gas sensor according to the present invention comprises: a solid electrolyte member, a sensing electrode provided on the solid electrolyte member and comprising at least one of a metal carbonate and a metal hydrogen carbonate; and a plurality of reference electrodes arranged on the solid electrolyte member so as to have mutually different impedances in the solid electrolyte member with respect to the sensing electrode.

According to the second gas sensor, the impedance between the reference electrode and the sensing electrode can be selected in accordance with a measurement application or an operation environment. Therefore, the second gas sensor can measure a gas concentration in various methods.

As described previously, the Inventors discovered that, in the case where a plurality of reference electrodes were provided on the solid electrolyte member and arranged so as to have their respective impedances different from each other in the solid electrolyte member between these reference electrodes and the sensing electrode, the reference electrodes had their respective degrees of impedance change relative to temperature change of the solid electrolyte member. Since the second gas sensor is able to detect the temperature of the solid electrolyte member on the basis of the difference of the electromotive forces at the respective reference electrodes, the temperature of the solid electrolyte member can be measured without use of the temperature detecting element. Since the temperature of the solid electrolyte member can be directly measured, the temperature of the solid electrolyte member can be measured with higher accuracy than with use of the temperature detecting element.

The second gas sensor may be configured so that the plurality of reference electrodes have their respective distances different from each other with respect to the sensing electrode. Alternatively, the second gas sensor may be configured so that the plurality of reference electrodes have their respective contact areas different from each other with respect to the solid electrolyte member. These configurations suitably realize the plurality of reference electrodes arranged so as to have mutually different impedances in the solid electrolyte member with respect to the sensing electrode.

The second gas sensor may be configured in a configuration comprising a plurality of switch means for switching a conducting state between two terminals, wherein one-side terminals of the respective switch means are electrically connected to the respective reference electrodes in one to one relation, and other-side terminals of the switch means are short-circuited with each other. This permits one of the reference electrodes to be suitably selected on the occasion of measuring the electromotive force, and thus achieves downsizing of a circuit for measuring the electromotive force between each of the reference electrodes and the sensing electrode.

The second gas sensor may be configured in a configuration further comprising amplifying means for amplifying a voltage between two input terminals, wherein one of the two input terminals of the amplifying means is electrically connected to the other-side terminals of the respective switch means, and wherein the other of the two input terminals of the amplifying means is electrically connected to the sensing electrode. This configuration permits the electromotive force to be measured with higher accuracy between each of the reference electrodes and the sensing electrode.

The second gas sensor preferably further comprises a heater for heating the solid electrolyte member.

The second gas sensor may be configured in a configuration further comprising temperature controlling means for obtaining a temperature of the solid electrolyte member on the basis of a difference of electromotive forces between each of the reference electrodes and the sensing electrode and for controlling a supplied power to the heater so that the obtained temperature nears a predetermined temperature. This configuration suitably realizes the gas sensor capable of accurately measuring the temperature of the solid electrolyte member without use of the temperature detecting element and easily and accurately controlling the temperature.

The second gas sensor may be configured in a configuration further comprising gas concentration calculating means for obtaining a temperature of the solid electrolyte member on the basis of a difference of electromotive forces between each of the reference electrodes and the sensing electrode and for obtaining a concentration of a measuring-object gas, based on a correlation between the electromotive force at at least one reference electrode and the concentration of the measuring-object gas, corresponding to the temperature. This configuration suitably realizes the gas sensor capable of accurately measuring the temperature of the solid electrolyte member without use of the temperature detecting element and easily and accurately measuring the concentration of the measuring-object gas.

The second gas sensor may be configured in a configuration further comprising gas concentration measuring means for measuring a concentration of a measuring-object gas, based on a correlation between an electromotive force between at least one reference electrode among the plurality of reference electrodes and sensing electrode, and the concentration of the measuring-object gas.

Another aspect of the present invention relates to a gas concentration measurement method. A first gas concentration measurement method according to the present invention is a gas concentration measurement method by use of the second gas sensor described above comprising: a selecting step of selecting a reference electrode from the plurality of reference electrodes; an electromotive force measuring step of measuring an electromotive force between the reference electrode selected in the selecting step, and the sensing electrode; and a gas concentration calculating step of obtaining a concentration of a measuring-object gas, based on a correlation between the electromotive force measured in the electromotive force measuring step and the concentration of the measuring-object gas.

The first gas concentration measurement method described above permits the impedance between the sensing electrode and the reference electrode to be changed according to a measurement application or an operation environment, by selecting one of the plurality of reference electrodes having mutually different impedances with respect to the sensing electrode.

A second gas concentration measurement method according to the present invention is a gas concentration measurement method by use of the first gas sensor described above comprising: a selecting step of selecting which electromotive force should be measured between a first electromotive force between one of the first and second reference electrodes and the sensing electrode and a second electromotive force between the first and second reference electrodes in a mutually short-circuited state of the first and second reference electrodes, and the sensing electrode; an electromotive force measuring step of measuring the first or second electromotive force selected in the selecting step; and a gas concentration calculating step of obtaining a concentration of a measuring-object gas, based on a correlation between the first or second electromotive force measured in the electromotive force measuring step and the concentration of the measuring-object gas.

The second gas concentration measurement method described above involves short-circuiting the first and second reference electrodes to enlarge the contact area between the reference electrode and the solid electrolyte member, so that the impedance between the reference electrode and the sensing electrode becomes smaller than in the case where the first or second reference electrode is singly used. Therefore, the above second gas concentration measurement method permits the impedance between the sensing electrode and the reference electrode to be changed according to a measurement application or an operation environment, by selecting which electromotive force should be measured between the first and second electromotive forces.

The first and second gas concentration measurement methods may further comprise a temperature selecting step of selecting a preset temperature of the solid electrolyte member; and a heating step of heating the solid electrolyte member so that a temperature of the solid electrolyte member nears the preset temperature, prior to the electromotive force measuring step. In the gas sensor, with increase in the temperature of the solid electrolyte member, the reaction is promoted between the metal carbonate or the metal hydrogen carbonate of the sensing electrode and the measuring-object gas. With decrease in the temperature of the solid electrolyte member, the amount of heat to the solid electrolyte member can be decreased, so as to reduce the power consumption. This gas concentration measurement method involves selecting the preset temperature of the solid electrolyte member, whereby activeness of reaction and power consumption can be changed according to a measurement application or an operation environment.

A third gas concentration measurement method according to the present invention is a gas concentration measurement method by use of the second gas sensor described above comprising: an electromotive force measuring step of measuring electromotive forces between the plurality of reference electrodes and the sensing electrode; and a gas concentration calculating step of obtaining a temperature of the solid electrolyte member on the basis of a difference between the electromotive forces, and obtaining a concentration of a measuring-object gas, based on a correlation between an electromotive force at at least one reference electrode among the plurality of reference electrodes and the concentration of the measuring-object gas, corresponding to the temperature.

The Inventors discovers that, in a case where a plurality of reference electrodes is provided on the solid electrolyte member and where the reference electrodes are arranged so as to have their respective impedances different from each other in the solid electrolyte member between these reference electrodes and the sensing electrode, the plurality of reference electrodes had their respective degrees of impedance change (change rates) relative to temperature change of the solid electrolyte member. Therefore, the temperature of the solid electrolyte member can be determined based on the difference of electromotive forces at the respective reference electrodes (e.g., an electromotive force difference). Since the third gas concentration measurement method described above involves obtaining the temperature of the solid electrolyte member on the basis of the difference of the electromotive forces at the respective reference electrodes, the temperature of the solid electrolyte member can be measured without use of the temperature detecting element. Since the temperature of the solid electrolyte member can be directly measured, the temperature of the solid electrolyte member can be measured with higher accuracy than with use of the temperature detecting element. This permits the concentration of the measuring-object gas to be obtained easily and accurately.

A fourth gas concentration measurement method according to the present invention is a gas concentration measurement method by use of the second gas sensor described above, comprising: an electromotive force measuring step of measuring electromotive forces between the plurality of reference electrodes and the sensing electrode; a temperature control step of obtaining a temperature of the solid electrolyte member on the basis of a difference between the electromotive forces, and controlling an amount of heat to the solid electrolyte member so that the temperature nears a predetermined temperature; and a gas concentration measuring step of obtaining a concentration of a measuring-object gas, based on a correlation between an electromotive force between at least one reference electrode out of the plurality of reference electrodes and the sensing electrode, and the concentration of the measuring-object gas, corresponding to the predetermined temperature.

In the fourth gas concentration measurement method described above, the temperature of the solid electrolyte member is obtained based on the difference of the electromotive forces at the respective reference electrodes in the same manner as in the third gas concentration measurement method, so that the temperature of the solid electrolyte member can be measured without use of the temperature detecting element. Since the temperature of the solid electrolyte member can be directly measured, the temperature of the solid electrolyte member can be measured with higher accuracy than with use of the temperature detecting element. This permits the temperature of the solid electrolyte member to be controlled easily and accurately.

A fifth gas concentration measurement method according to the present invention is a gas concentration measurement method by use of the first gas sensor described above, comprising: an electromotive force measuring step of measuring a first electromotive force between the sensing electrode and either one of first and second reference electrodes, and measuring a second electromotive force between the first and second reference electrodes and the sensing electrode in a short-circuited state of the first and second reference electrodes; and a gas concentration calculating step of obtaining a temperature of the solid electrolyte member on the basis of a difference between the first electromotive force and the second electromotive force and obtaining a concentration of a measuring-object gas, based on a correlation between the first or second electromotive force and the concentration of the measuring-object gas corresponding to the temperature.

In the fifth gas concentration measurement method described above, the electromotive force between one of the two reference electrodes and the sensing electrode (first electromotive force) is measured, and the electromotive force between the reference electrodes and the sensing electrode (second electromotive force) is further measured in the short-circuited state of the two reference electrodes (i.e., in a state in which the contact area is enlarged between the solid electrolyte member and the reference electrodes). By changing the contact area between the reference electrode and the solid electrolyte member in this manner, the impedance can be suitably changed between the reference electrode and the sensing electrode. Therefore, the temperature of the solid electrolyte member can be determined based on the difference between the first electromotive force and the second electromotive force (e.g., an electromotive force difference). Since the above-described fifth gas concentration measurement method involves obtaining the temperature of the solid electrolyte member on the basis of the difference between the first electromotive force and the second electromotive force, the temperature of the solid electrolyte member can be measured without use of the temperature detecting element. In addition, since the temperature of the solid electrolyte member can be directly measured, the temperature of the solid electrolyte member can be measured with higher accuracy than with use of the temperature detecting element. This permits the concentration of the measuring-object gas to be obtained easily and accurately.

A sixth gas concentration measurement method according to the present invention is a gas concentration measurement method by use of the first gas sensor described above, comprising: an electromotive force measuring step of measuring a first electromotive force between one of the first and second reference electrodes and the sensing electrode, and measuring a second electromotive force between the first and second reference electrodes and the sensing electrode in a short-circuited state of the first and second reference electrodes; a temperature control step of obtaining a temperature of the solid electrolyte member on the basis of a difference between the first electromotive force and the second electromotive force and controlling an amount of heat to the solid electrolyte member so that the temperature nears a predetermined temperature; and a gas concentration measuring step of measuring a third electromotive force between at least one of the first and second reference electrodes and the sensing electrode, and obtaining a concentration of a measuring-object gas, based on a correlation between the third electromotive force and the concentration of the measuring object gas corresponding to the predetermined temperature.

In the sixth gas concentration measurement method described above, the temperature of the solid electrolyte member is obtained based on the difference between the first electromotive force and the second electromotive force in the same manner as in the fifth gas concentration measurement method, and thus the temperature of the solid electrolyte member can be determined without use of the temperature detecting element Since the temperature of the solid electrolyte member can be directly measured, the temperature of the solid electrolyte member can be measured with higher accuracy than with use of the temperature detecting element. This permits the temperature of the solid electrolyte member to be controlled easily and accurately.

The third to sixth gas concentration measurement methods preferably further comprise a step of heating the solid electrolyte member, prior to the electromotive force measuring step.

### BRIEF DESCRIPTION OF THE DRAWINGS

Fig. 1 is a schematic diagram showing a configuration of the first embodiment of the gas sensor according to the present invention.
Fig. 2 is a sectional view showing cross section I-I of the sensor element shown in Fig. 1.
Fig. 3 is a flowchart showing the operation of the gas sensor according to the first embodiment.
Fig. 4 is a plan view showing a configuration of a modification example of the gas sensor according to the first embodiment.
Fig. 5 is a schematic diagram showing a configuration of a gas sensor according to the second embodiment.
Fig. 6 is a flowchart showing the operation of the gas sensor according to the second embodiment.
Fig. 7 is a schematic diagram showing a configuration of the third embodiment of the gas sensor according to the present invention.
Fig. 8 is a perspective view of a device experimentally produced in order to find out the correlation between the difference of electromotive forces between each of a plurality of reference electrodes and a sensing electrode, and temperature of a solid electrolyte member.
Fig. 9 is a graph showing changes of impedances between each pair of electrodes.
Fig. 10 is a flowchart showing the operation of the gas sensor in the third embodiment.
Fig. 11 is a schematic diagram showing a configuration of a gas sensor according to the fourth embodiment.
Fig. 12 is a flowchart showing the operation of the gas sensor according to the fourth embodiment.
Fig. 13 is a schematic diagram showing a configuration of a gas sensor according to the fifth embodiment.
Fig. 14 is a flowchart showing the operation of the gas sensor according to the fifth embodiment.
Fig. 15 is a schematic diagram showing a configuration of a gas sensor according to the sixth embodiment.
Fig. 16 is a flowchart showing the operation of the gas sensor according to the sixth embodiment.

### DESCRIPTION OF THE PREFERRED EMBODIMENTS

Embodiments of the gas concentration measurement method and gas sensor according to the present invention will be described below in detail with reference to the accompanying drawings. Identical elements will be denoted by the same reference symbols in the description of the drawings, without redundant description.

### (First Embodiment)

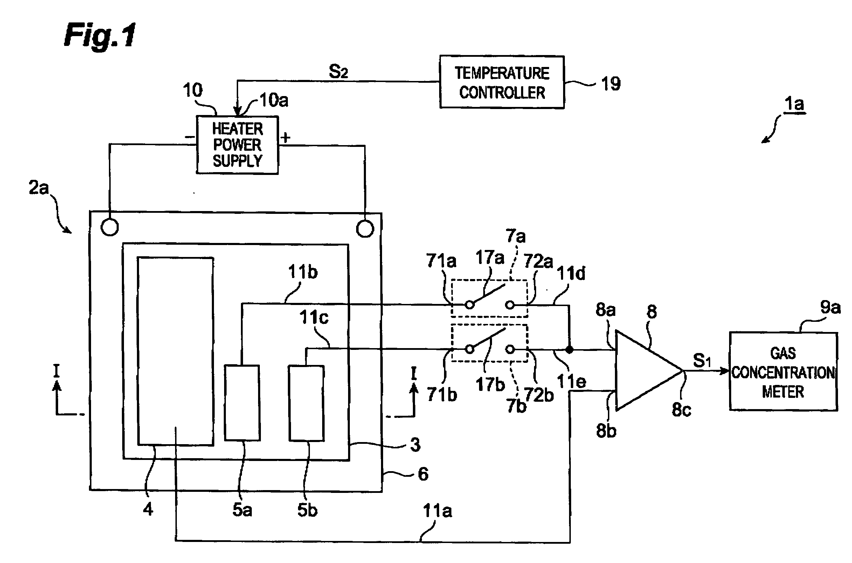

Fig. 1 is a schematic diagram showing a configuration of the first embodiment of the gas sensor according to the present invention. The gas sensor 1a in the first embodiment can be used, for example, as a carbon dioxide sensor the measuring-object gas of which is carbon dioxide.

The gas sensor 1a is provided with sensor element 2a, a plurality of switches (switch means) 7a and 7b, amplifier 8, gas concentration meter (gas concentration measuring means) 9a, heater power supply 10, wires 11a-11e, and a temperature controller (temperature controlling means) 19.

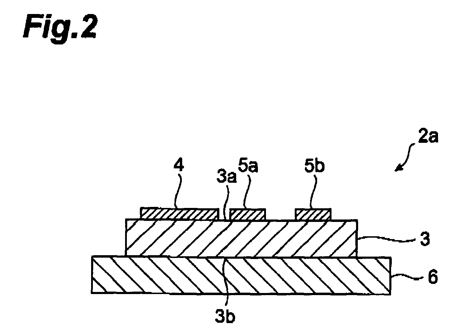

The sensor element 2a is a device that generates an electromotive force according to a concentration of a measuring-object gas. Fig. 2 is a sectional view showing cross section I-I of the sensor element 2a shown in Fig. 1. Referring to Fig. 2, the sensor element 2a has solid electrolyte member 3 of flat plate shape, sensing electrode 4 provided in contact on one surface 3a of the solid electrolyte member 3, a plurality of reference electrodes 5a and 5b arranged in contact on one surface 3a of the solid electrolyte member 3 and apart from the sensing electrode 4, and heater 6 arranged in contact on another surface 3b of the solid electrolyte member 3.

The solid electrolyte member (member containing a solid electrolyte) 3 is a metal ion conductor and can be, for example, an alkali metal ion and/or alkali earth metal ion conductor, preferably, a sodium ion conductor. Such ion conductors are preferably NASICON (specifically, Na₃Zr₂Si₂PO₁₂ or the like) represented by Na₁₊ₓZr₂SiₓP₃₋ₓO₁₂ (x = 0 to 3), for example.

The solid electrolyte member 3 may contain a reinforcing agent in an amount not impeding ion conduction, in addition to the metal ion conductor; for example, it may contain 50% or less in terms of percentage by mass of aluminum oxide (Al₂O₃, silicon oxide (SiO₂), zirconium oxide (ZrO₂), silicon carbide (SiC), silicon nitride (Si₃N₄), iron oxide (Fe₂O₃), or the like.

The sensing electrode 4 preferably contains a metal carbonate and/or a metal hydrogen carbonate in the case where carbon dioxide is measured as a measuring-object gas. When the sensing electrode 4 contains the metal carbonate and/or the metal hydrogen carbonate, it further promotes generation of hydrogen carbonate ions essential to detection of carbon dioxide and further improves sensitivity, response speed, selectivity, and so on. A conceivable reason for it is that the metal carbonate reacts with carbon dioxide and water to make a metal hydrogen carbonate, thereby promoting generation of hydrogen carbonate ions originating from carbon dioxide.

The metal carbonates available herein include, for example, lithium carbonate (Li₂CO₃), sodium carbonate (Na₂CO₃), barium carbonate (BaCO₃), and so on. The metal hydrogen carbonates available herein include, for example, hydrogen carbonates of alkali metals, such as sodium hydrogen carbonate (NaHCO₃), potassium hydrogen carbonate (KHCO₃), rubidium hydrogen carbonate (RbHCO₃), cesium hydrogen carbonate (CsHCO₃), and so on.

The sensing electrode 4 may contain a metal oxide. The metal oxide included in the sensing electrode 4 preferably has electron conductivity and is preferably indium oxide (In₂O₃), for example.

The reference electrodes 5a and 5b are located each a predetermined distance apart from the sensing electrode 4 and formed in contact on the solid electrolyte member 3. The reference electrodes 5a and 5b are arranged so as to have their respective impedances different from each other in the solid electrolyte member 3 with respect to the sensing electrode 4. In the present embodiment, the distance between reference electrode 5a and sensing electrode 4 is shorter than the distance between reference electrode 5b and sensing electrode 4, whereby the impedance between reference electrode 5a and sensing electrode 4 is smaller than the impedance between reference electrode 5b and sensing electrode 4. The material of the reference electrodes 5a and 5b is preferably a metal or an electrically conductive metal oxide or the like.

The heater 6 is provided in contact on the other surface 3b of the solid electrolyte member 3. The heater 6 is electrically connected to the heater power supply 10 (cf. Fig. 1), and it generates heat with supply of power from the heater power supply 10 and supplies the heat to the solid electrolyte member 3. The solid electrolyte member 3 is heated at a temperature suitable for ion conduction (approximately 350°C or higher in the case of NASICON) by the heater 6.

Reference is made again to Fig. 1. The temperature controller 19 is a means for controlling the supplied power to the heater 6 so that the temperature of the solid electrolyte member 3 nears the preset temperature. The temperature controller 19 can be realized in such a manner that an arithmetic operation device such as a CPU executes a predetermined program stored in a storage device such as a hard disk or a memory, for example. The preset temperature of the solid electrolyte member 3 may be set at an arbitrary temperature by a measuring person or may be selected among a preset temperature group by a measuring person. Where the solid electrolyte member 3 contains NASICON, the preferred value of the preset temperature is, for example, 150°C-700°C. The temperature controller 19 is electrically connected to control terminal 10a for controlling the output of heater power supply 10 and provides a power control signal S₂ for controlling the supplied power to the heater 6 according to the preset temperature, to the heater power supply 10. The gas sensor 1a may be provided with a temperature detecting element such as a thermistor or a thermocouple not shown, in order to control the temperature of the solid electrolyte member 3. In this case, the temperature controller 19 generates the power control signal S₂ so that the temperature of the solid electrolyte member 3 nears the preset temperature, based on a temperature signal from the temperature detecting element.

The switch 7a has a pair of terminals 71a and 72a. The switch 7b has a pair of terminals 71b and 72b. The switches 7a and 7b are arranged so as to be able to switch a conducting state between terminal 71a and terminal 72a and a conducting state between terminal 71b and terminal 72b, respectively The switches 7a and 7b are used for selecting (or switching) either of the reference electrodes 5a and 5b. The one-side terminals 71a and 71b of the switches 7a and 7b are electrically connected through respective wires 11b and 11c to the reference electrodes 5a and 5b, respectively. The other-side terminals 72a and 72b of the switches 7a and 7b are short-circuited with each other through wires 11d and 11e. The switches 7a and 7b of the present embodiment are realized by mechanical switches 17a and 17b, and the switches 7a and 7b may be realized by semiconductor switches such as transistors, for example.

The amplifier 8 is an amplifying means for amplifying a voltage between two input terminals 8a and 8b. One input terminal 8a of the amplifier 8 is electrically connected through wires 11d and 11e to the terminals 72a and 72b of switches 7a and 7b. The other input terminal 8b of the amplifier 8 is electrically connected through wire 11a to sensing electrode 4. An output terminal 8c of the amplifier 8 is electrically connected to gas concentration calculator 9a. In this configuration, when the switch 7a or 7b is brought into a conducting state, the amplifier 8 amplifies a potential difference (electromotive force) between the reference electrode 5a or 5b and the sensing electrode 4 to generate an electromotive force signal S₁, and provides this electromotive force signal S₁ to the gas concentration calculator 9a.

The gas concentration meter 9a is a means for measuring the electromotive force between the reference electrode 5a or 5b selected by the switch 7a or 7b, and the sensing electrode 4 and for obtaining a concentration of the measuring-object gas, based on a correlation between the electromotive force and the concentration of the measuring-object gas. The gas concentration meter 9a is preferably configured to preliminarily store correlation data between the electromotive force at each of the reference electrodes 5a and 5b and the carbon dioxide concentration at each temperature and to apply the electromotive force value between the reference electrode 5a or 5b and the sensing electrode 4 and the preset temperature of the solid electrolyte member 3 to this correlation data, thereby obtaining the carbon dioxide concentration. The gas concentration meter 9a can be realized in such a manner that an arithmetic operation device such as a CPU storing a map of characteristic values prepared in advance executes an arithmetic operation according to a predetermined program, for example. The map of characteristic values and the program may be used as stored in an external storage device such as a hard disk or a memory, for example.

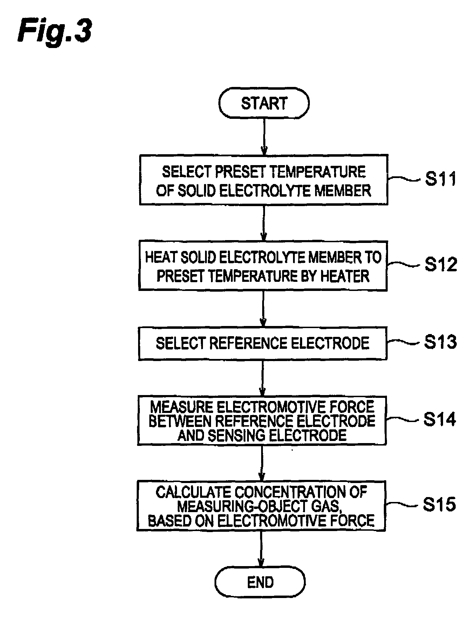

The above described the configuration of gas sensor 1a according to the present embodiment. Subsequently, a gas concentration measurement method according to the present embodiment will be described together with the operation of gas sensor 1a with reference to Fig. 3. Fig. 3 is a flowchart of the gas concentration measurement method including the operation of the gas sensor 1a. In the description below, the operation and method will be described using carbon dioxide as the measuring-object gas and lithium carbonate as the material included in the sensing electrode 4.

First, the preset temperature of the solid electrolyte member 3 is selected and the preset temperature is fed to the temperature controller 19 (temperature selecting step, S11). The temperature controller 19 controls the supplied power from the heater power supply 10 to the heater 6 in accordance with the selected preset temperature. The heater 6 heats the solid electrolyte member 3 to the preset temperature (heating step, S12). This results in changing lithium carbonate in the sensing electrode 4 into an active state as Li₂CO₃ is separated and ionized into 2Li⁺ and CO₃²⁻. 2Li⁺ react with oxygen (O₂) or carbon dioxide (CO₂) entering the sensing electrode 4 from the outside, as indicated by the reaction of (1) below, to form new Li₂CO₃.

CO₂ + 2Li⁺ + 1/2O₂ + 2e⁻ → Li₂CO₃ (1)

Li₂CO₃ newly formed reacts with Na ions being conductive ions in the solid electrolyte member 3, as indicated by the reaction of (2) below.

Li₂CO₃ + 2Na⁺ → CO₂ + 2Li⁺ + 1/2O₂ + 2e⁻ + 2Na⁺ → 2Li⁺ + Na₂CO₃ (2)

In the above reaction, Li₂CO₃ releases electrons in the reaction with the Na ions. The electrons thus released are taken into Na₂CO₃ to induce an electromotive force between the sensing electrode 4 and the reference electrodes 5a and 5b.

Subsequently, either of the reference electrodes 5a and 5b is selected (selecting step, S13). Namely, one of the switches 7a and 7b is closed (to be brought into a conducting state), and the other is opened (to be brought into a non-conducting state). This results in feeding the electromotive force between the selected reference electrode 5a or 5b and the sensing electrode 4 to the input terminals 8a and 8b of the amplifier 8, and the amplifier 8 amplifies the electromotive force to produce an electromotive force signal S₁, and provides the signal to the gas concentration meter 9a (electromotive force measuring step, S14). The gas concentration meter 9a obtains the carbon dioxide concentration, based on the correlation between the electromotive force signal S₁ and the carbon dioxide concentration (gas concentration calculating step, S15).

The gas concentration measurement method and gas sensor 1a of the present embodiment described above involve selecting one reference electrode 5a or 5b out of the plurality of reference electrodes 5a and 5b with mutually different impedances with respect to the sensing electrode 4, whereby they can change the impedance between sensing electrode 4 and the reference electrode according to a measurement application or an operation environment.

In the gas concentration measurement method and gas sensor 1a of the present embodiment, the plurality of reference electrodes 5a and 5b preferably have their respective distances different from each other with respect to the sensing electrode 4. This permits the reference electrodes 5a and 5b to be suitably arranged so as to have their respective impedances different from each other in the solid electrolyte member 3 with respect to the sensing electrode 4.

The gas sensor 1a preferably comprises an amplifying means such as the amplifier 8 as in the present embodiment. This permits the electromotive force between the reference electrode 5a or 5b and the sensing electrode 4 to be measured with higher accuracy.

The gas sensor 1a preferably comprises a plurality of switches 7a and 7b according to the plurality of reference electrodes 5a and 5b as in the present embodiment. This permits the gas concentration meter 9a to suitably select one of the plurality of reference electrodes 5a and 5b in the measurement of the electromotive force.

The gas concentration measurement method preferably comprises the temperature selecting step S11 of selecting the preset temperature of the solid electrolyte member 3, and the heating step S12 of heating the solid electrolyte member 3 so that the temperature of the solid electrolyte member 3 nears the preset temperature, prior to the electromotive force measuring steps S14 and S15, as in the present embodiment. The gas sensor 1a preferably comprises the heater 6 for heating the solid electrolyte member 3, and the temperature controller 19 for controlling the supplied power to the heater 6 so that the temperature of the solid electrolyte member 3 nears the preset temperature. In the gas sensor 1a of the present embodiment, with increase in the temperature of the solid electrolyte member 3, the reaction is promoted between the metal carbonate or the metal hydrogen carbonate of the sensing electrode 4 and the measuring-object gas. With decrease in the temperature of the solid electrolyte member 3, the amount of heat to the solid electrolyte member 3 can be small, so as to reduce the power consumption. Therefore, activeness of reaction and power consumption can be suitably changed according to a measurement application or an operation environment, by selecting the preset temperature of the solid electrolyte member 3.

An example of operation modes realized by the gas concentration measurement method and gas sensor 1a according to the present embodiment will be described below.

### (1) Low Power Consumption Mode

This operation mode is one in which the temperature selecting step S11 is to set the preset temperature of the solid electrolyte member 3 at a relatively low temperature (e.g., 150°C-250°C) and in which the selecting step S13 is to select the reference electrode 5b with the larger impedance with respect to the sensing electrode 4. In this operation mode, the solid electrolyte member 3 is set at the low temperature to decrease the reaction rate between the metal carbonate or the metal hydrogen carbonate of the sensing electrode 4 and the measuring-object gas, but the impedance is large between the reference electrode 5b and the sensing electrode 4; therefore, the electromotive force can be maintained even by a reduced amount of conductive ions, and the concentration of the measuring-object gas can be suitably measured. Therefore, the power consumption in the heater 6 can be controlled at a low level in this operation mode.

### (2) High Concentration Measurement Mode

This operation mode is one in which the temperature selecting step S11 is to set the preset temperature of the solid electrolyte member 3 at a relatively high temperature (e.g., 300°C-450°C) and in which the selecting step S13 is to select the reference electrode 5a with the smaller impedance with respect to the sensing electrode 4. In this operation mode, the solid electrolyte member 3 is set at the high temperature and thus the reaction is promoted between the metal carbonate or the metal hydrogen carbonate of the sensing electrode 4 and the measuring-object gas. The impedance is small between the reference electrode 5a and the sensing electrode 4 to increase the response speed from generation of conductive ions to measurement thereof as an electromotive force. In this operation mode, therefore, the concentration of the measuring-object gas can be obtained faster, and can be measured without saturation even in cases where the concentration of the measuring-object gas is relatively high.

### (3) Simplified Measurement Mode

This operation mode is one in which the temperature selecting step S11 is to set the preset temperature of the solid electrolyte member 3 at a relatively low temperature (e.g., 150°C-250°C) and in which the selecting step S13 is to select the reference electrode 5a with the smaller impedance with respect to the sensing electrode 4. In this operation mode, the solid electrolyte member 3 is set at the low temperature and thus the reaction rate is decreased between the metal carbonate or the metal hydrogen carbonate of the sensing electrode 4 and the measuring-object gas. However, the impedance is small between the reference electrode 5a and the sensing electrode 4, and thus the response speed is high from generation of conductive ions to measurement thereof as an electromotive force. Therefore, this operation mode is suitable for applications to simply measure the measuring-object gas at a relatively high concentration and with considerable concentration change, while keeping the power consumption in the heater 6 at a low level.

### (4) High-accuracy Measurement Mode

This operation mode is one in which the temperature selecting step S11 is to set the preset temperature of the solid electrolyte member 3 at a relatively high temperature (e.g., 300°C-450°C) and in which the selecting step S13 is to select the reference electrode 5b with the larger impedance with respect to the sensing electrode 4. In this operation mode, the solid electrolyte member 3 is set at the high temperature and the reaction is promoted between the metal carbonate or the metal hydrogen carbonate of the sensing electrode 4 and the measuring-object gas. Since the impedance is large between the reference electrode 5b and the sensing electrode 4, the electromotive force can be maintained even by a reduced amount of conductive ion. Therefore, the concentration can be accurately measured even in cases where the concentration of the measuring-object gas is relatively low.

### (Modification Example)

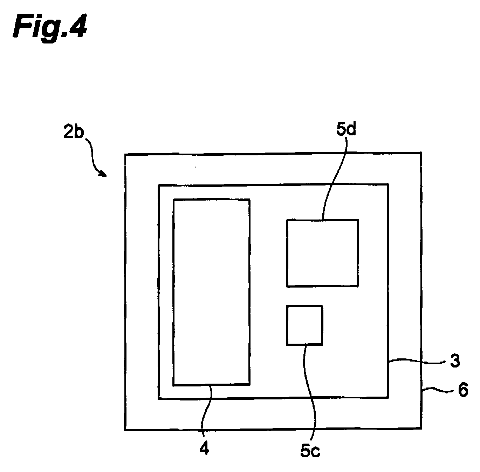

Fig. 4 is a plan view showing a configuration of sensor element 2b as a modification example of the gas sensor 1a according to the first embodiment. The gas sensor 1a of the first embodiment may comprise the sensor element 2b of the present modification example instead of the sensor element 2a. The sensor element 2b of the present modification example is provided with solid electrolyte member 3, sensing electrode 4, a plurality of reference electrodes 5c, and 5d, and heater 6. Among these, the components other than the plurality of reference electrodes 5c and 5d are constructed in much the same configuration as in the first embodiment and thus the description thereof is omitted herein.

The reference electrodes 5c and 5d in the present modification example have their respective contact areas different from each other with respect to the solid electrolyte member 3. Specifically, the reference electrode 5d is formed in a larger area than the reference electrode 5c. The reference electrode 5c and the reference electrode 5d have their respective distances approximately equal to each other with respect to the sensing electrode 4. The plurality of reference electrodes 5c, 5d with mutually different impedances in the solid electrolyte member 3 with respect to the sensing electrode 4 can also be suitably realized by arranging the reference electrodes 5c, 5d so as to have their respective contact areas different from each other with respect to the solid electrolyte member 3 as in the present modification example.

### (Second Embodiment)

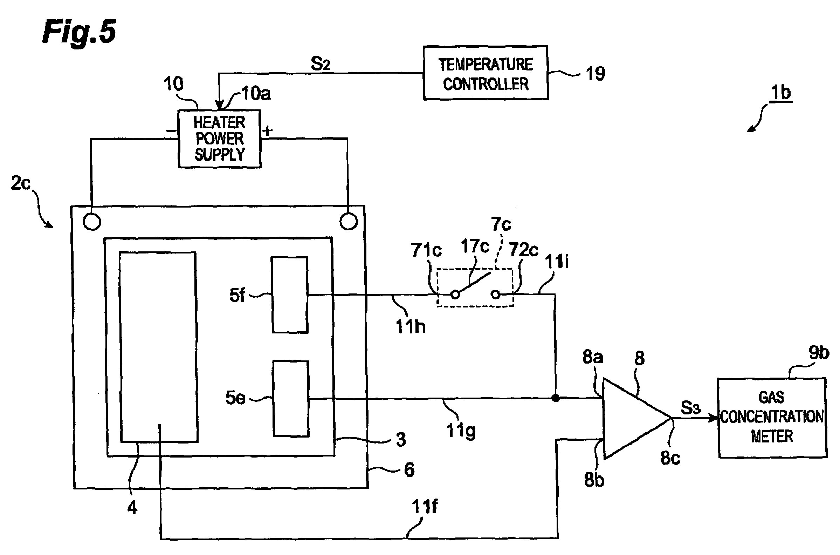

Subsequently, the second embodiment of the gas concentration measurement method and the gas sensor according to the present invention will be described. Fig. 5 is a schematic diagram showing a configuration of gas sensor 1b according to the present embodiment. The gas sensor 1b is provided with sensor element 2c, switch 7c, amplifier 8, gas concentration meter 9b, heater power supply 10, wires 11f-11i, and temperature controller 19. Among these components, the heater power supply 10 and temperature controller 19 are constructed in much the same configuration as in the first embodiment, and the description thereof is omitted herein.

The sensor element 2c is a device that generates an electromotive force according to a concentration of a measuring-object gas. The sensor element 2c of the present embodiment is different in the arrangement of the reference electrode 5e (first reference electrode) and the reference electrode 5f (second reference electrode) from the sensor element 2a of the first embodiment. In the present embodiment, the reference electrodes 5e and 5f are located at their respective distances approximately equal to each other with respect to the sensing electrode 4 on the solid electrolyte member 3 and are also formed in their respective contact areas approximately equal to each other with respect to the solid electrolyte member 3. Namely, the reference electrodes 5e and 5f have their respective impedances approximately equal to each other in the solid electrolyte member 3 with respect to the sensing electrode 4. Although the present embodiment involves the above-described arrangement and shape of the reference electrodes 5e and 5f in order to clearly show the difference from the first embodiment, the first and second reference electrodes of the present invention may have their respective distances different from each other with respect to the sensing electrode and may have their respective contact areas different from each other with respect to the solid electrolyte member 3. The solid electrolyte member 3, sensing electrode 4, and heater 6 are constructed in much the same configuration as in the first embodiment.

The switch 7c is a means for short-circuiting the reference electrodes 5e and 5f with each other. The switch 7c has a pair of terminals 71c and 72c and is able to switch a conducting state between terminal 71c and terminal 72c. One terminal 71c of the switch 7c is electrically connected through wire 11h to the reference electrode 5f. The other terminal 72c of switch 7c is electrically connected through wires 11g and 11i to the reference electrode 5e. The switch 7c of the present embodiment is realized by mechanical switch 17c as the switches 7a and 7b of the first embodiment were, but the switch 7c may be realized by a semiconductor switch such as a transistor, for example.

One input terminal 8a of the amplifier 8 is electrically connected through wire 11i to terminal 72c of the switch 7c and electrically connected through wire 11g to the reference electrode 5e. The other input terminal 8b of the amplifier 8 is electrically connected through wire 11f to the sensing electrode 4. The output terminal 8c of the amplifier 8 is electrically connected to the gas concentration meter 9b. In this configuration, while the switch 7c is in a non-conducting state, the amplifier 8 amplifies a potential difference (electromotive force) between reference electrode 5e and sensing electrode 4 to generate an electromotive force signal S₃. While the switch 7c is in a conducting state, the amplifier 8 amplifies a potential difference (electromotive force) between both of the reference electrodes 5e and 5f (i.e., enlarged reference electrode) and the sensing electrode 4 to generate an electromotive force signal S₃. The amplifier 8 provides the generated electromotive force signal S₃ for the gas concentration meter 9b.

The gas concentration meter 9b is a means for measuring the electromotive force between reference electrode 5e and sensing electrode 4 (first electromotive force), or the electromotive force between the reference electrodes 5e and 5f short-circuited by the switch 7c, and the sensing electrode 4 (second electromotive force), and for obtaining a concentration of the measuring-object gas, based on a correlation between the first or second electromotive force and the concentration of the measuring-object gas. The gas concentration meter 9b is preferably configured to preliminarily store correlation data of carbon dioxide concentration with the electromotive force at the single reference electrode 5e and the electromotive force in the mutually short-circuited state of the reference electrodes 5e and 5f at each temperature, and to apply the first or second electromotive force value measured and the preset temperature of the solid electrolyte member 3 to this correlation data, thereby obtaining the carbon dioxide concentration. The gas concentration meter 9b can be realized in such a way that an arithmetic operation device such as a CPU storing a map of characteristics prepared in advance executes an arithmetic operation according to a predetermined program. The map of characteristic values and the program may be used as stored in an external storage device such as a hard disk or a memory, for example.

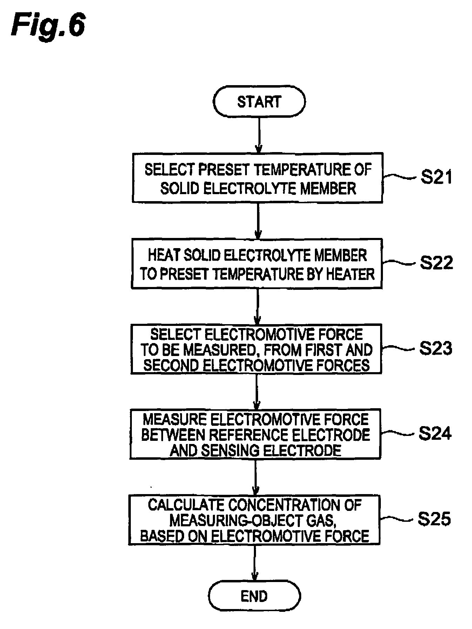

Subsequently, a gas concentration measurement method according to the present embodiment will be described together with the operation of gas sensor 1b with reference to Fig. 6. Fig. 6 is a flowchart showing the gas concentration measurement method including the operation of gas sensor 1b.

First, the preset temperature of the solid electrolyte member 3 is selected and the preset temperature is fed to the temperature controller 19 (temperature selecting step, S21). The temperature controller 19 controls the supplied power from heater power supply 10 to heater 6 in accordance with the preset temperature thus selected. The heater 6 heats the solid electrolyte member 3 to the preset temperature (heating step, S22). This results in inducting an electromotive force according to a carbon dioxide concentration between the sensing electrode 4 and the reference electrodes 5e and 5f

Subsequently, the electromotive force to be measured is selected from the first electromotive force between reference electrode 5e and sensing electrode 4, and the second electromotive force between the reference electrodes 5e and 5f in the mutually short-circuited state of the reference electrodes 5e and 5f, and the sensing electrode 4 (selecting step, S23). Where the first electromotive force is selected, the switch 7c is opened (to be brought into a non-conducting state). Where the second electromotive force is selected, the switch 7c is closed (to be brought into a conducting state). This results in feeding the first or second electromotive force to the input terminals 8a and 8b of the amplifier 8, and the amplifier 8 amplifies the electromotive force to produce the electromotive force signal S₃ and provides the signal to the gas concentration meter 9b (electromotive force measuring step, S24). The gas concentration meter 9b obtains the carbon dioxide concentration, based on the correlation between the electromotive force signal S₃ and the carbon dioxide concentration (gas concentration calculating step, S25).

The gas concentration measurement method and gas sensor 1b according to the present embodiment have the following effect. Namely, the gas concentration measurement method and gas sensor 1b according to the present embodiment involve short-circuiting the reference electrodes 5e and 5f to enlarge the contact area between the reference electrode and the solid electrolyte member 3, whereby the impedance between the reference electrodes 5e and 5f and the sensing electrode 4 becomes smaller than with single use of the reference electrode 5e or 5f. In the gas concentration measurement method and gas sensor 1b of the present embodiment, therefore, the electromotive force to be measured is selected from the first electromotive force between reference electrode 5e and sensing electrode 4 and the second electromotive force between the reference electrodes 5e and 5f in the mutually short-circuited state of the reference electrodes 5e and 5f, and the sensing electrode 4, whereby the impedance between sensing electrode 4 and the reference electrode can be suitably changed according to a measurement application or an operation environment.

The gas concentration measurement method and gas sensor 1b according to the present embodiment are able to suitably realize each of the operation modes described in the first embodiment (low power consumption mode, high concentration measurement mode, simplified measurement mode, and high-accuracy measurement mode).

The gas concentration measurement methods and gas sensors according to the present invention are not limited to the above embodiments, but may be modified in various ways. For example, each of the above embodiments was arranged to make, for example, a measuring person select the impedance value between the sensing electrode and the reference electrode, but the sensor may be provided with a selector (selecting means) for automatically performing this selection. Namely, for example, when the operator enters each of the operation modes described in the first embodiment, the selector selects the suitable impedance value in the entered operation mode to switch the switch means. Concerning the selection of the preset temperature of the solid electrolyte member, the gas sensor may be provided with a temperature selector (temperature selecting means) for automatically performing the selection of the preset temperature.

The gas concentration measurement methods of the respective embodiments were arranged to perform the temperature selecting step, the heating step, and the selecting step in the order named, but the selecting step may be carried out before the heating step, or before the temperature selecting step. In another arrangement, the selecting step and the temperature selecting step may be carried out simultaneously.

Each of the above embodiments is arranged to measure carbon dioxide as a measuring-object gas. The measuring-object gas in the present invention can be any gas the concentration of which can be measured based on the electromotive force in the solid electrolyte member, and the present invention can also be applied, for example, to oxygen sensors and others.

In each of the above embodiments, the gas sensor is provided with the solid electrolyte member of flat plate shape. The solid electrolyte member is not limited to the flat plate shape, but can be formed in other various shapes.

In each of the above embodiments, the gas sensor is provided with two reference electrodes. The number of reference electrodes is not limited to two, but may be three or more. In this case, one reference electrode can be selected from three or more reference electrodes (or the number of combinations for short-circuiting the reference electrodes with each other increases), whereby the impedance between the reference electrode and the sensing electrode can be changed finer.

In each of the above embodiments, the sensing electrode and the plurality of reference electrodes are provided each on the same surface of the solid electrolyte member, but the sensing electrode and the plurality of reference electrodes may be provided on their respective surfaces different from each other. The plurality of reference electrodes may also be provided on their respective surfaces different from each other.

### (Third Embodiment)

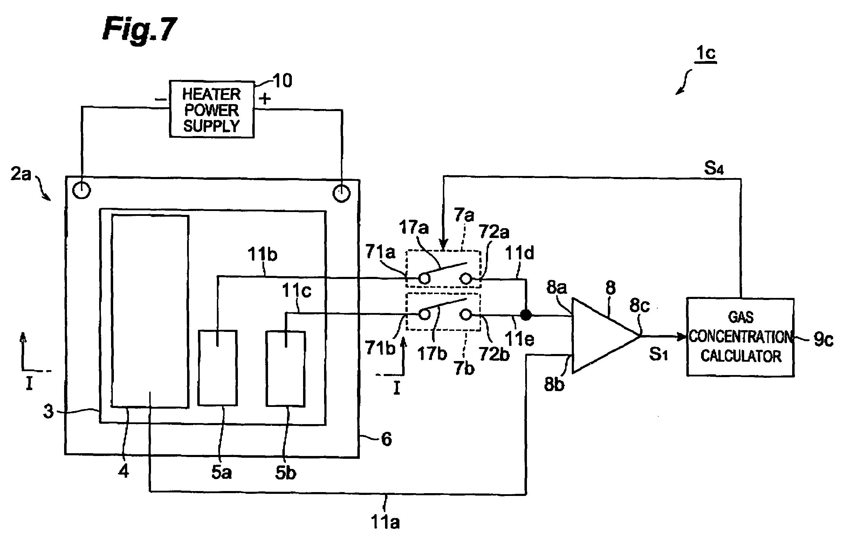

Fig. 7 is a schematic diagram showing a configuration of the third embodiment of the gas sensor according to the present invention. The gas sensor 1c in the third embodiment can be used, for example, as a carbon dioxide sensor the measuring-object gas of which is carbon dioxide.

The gas sensor 1c is provided with sensor element 2a, a plurality of switches (switch means) 7a and 7b, amplifier 8, gas concentration calculator 9c, heater power supply 10, and wires 11a-11e. Among these components, the sensor element 2a, the plurality of switches 7a and 7b, amplifier 8, and wires lla-lle are constructed in much the same configuration as in the first embodiment, and the description thereof is omitted herein. In addition, the heater power supply 10 is different from the heat power supply 10 of the first embodiment in the configuration in which the temperature controller 19 is not connected to the heat power supply 10 of the present embodiment. Each of the switches 7a and 7b has a control terminal (not shown) for controlling the conducting states thereof.

The gas concentration calculator 9c is a means for obtaining the temperature of the solid electrolyte member 3 on the basis of the difference of electromotive forces between each of the reference electrodes 5a and 5b and the sensing electrode 4 and for obtaining a concentration of the measuring-object gas, based on a correlation between the electromotive force and the concentration of the measuring-object gas corresponding to the temperature. The gas concentration calculator 9c can be realized, for example, in such a manner that an arithmetic operation device such as a CPU storing a map of characteristic values prepared in advance executes an arithmetic operation according to a predetermined program. The map of characteristic values and the program may be stored in an external storage, for example, such as a hard disk or a memory.

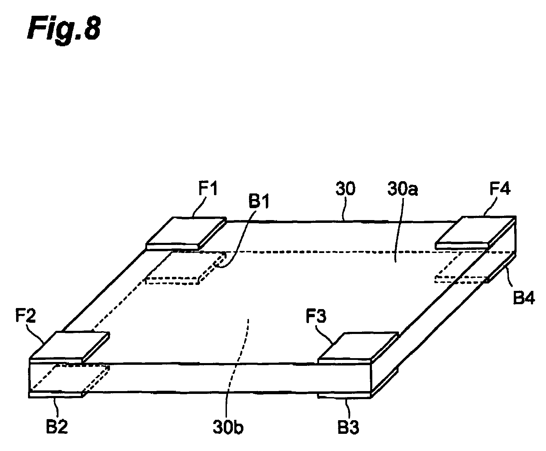

Described below is the correlation between the difference of electromotive forces between each of the reference electrodes and the sensing electrode, and the temperature of the solid electrolyte member. Fig. 8 is a perspective view of a device experimentally produced in order to find out this correlation. In this device, electrodes F1-F4 are located at the four corners on front face 30a of solid electrolyte member 30 of rectangular plate shape made of NASICON, and electrodes B1-B4 are located at the four corners on back face 30b. Namely, the distance between electrode F1 and electrode B1 agrees with the thickness of the solid electrolyte member 30, the distance between electrode F1 and electrode F2 agrees with the length of one side of front face 30a of solid electrolyte member 30, the distance between electrode F1 and electrode F3 agrees with the length of a diagonal line of front face 30a of the solid electrolyte member 30, and the distance between electrode F1 and electrode B3 agrees with the length of a diagonal line from front face 30a to back face 30b of solid electrolyte member 30. There is the relationship of (distance between electrode F1 and electrode B1) < (distance between electrode F1 and electrode F2) < (distance between electrode F1 and electrode F3) < (distance between electrode F1 and electrode B3), and the impedances between the electrodes also increase in this order.

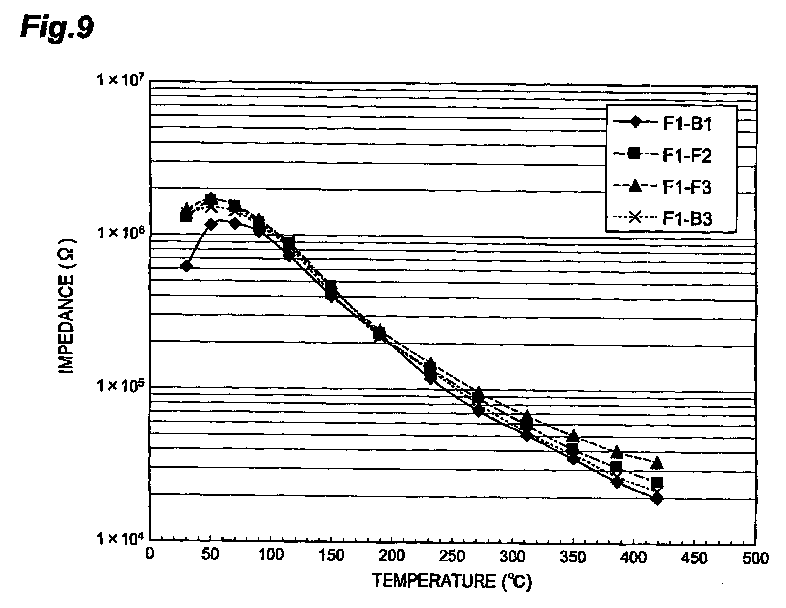

In the device shown in Fig. 8, changes of impedances between electrodes were measured relative to temperature change of the solid electrolyte member 30, while heating the solid electrolyte member 30. Fig. 9 is a graph showing changes of impedances between electrode F1 and electrode B1, between electrode F1 and electrode F2, between electrode F1 and electrode F3, and between electrode F1 and electrode B3. As shown in this graph, the impedances between the electrodes decrease with increase in the temperature of the solid electrolyte member 30. Then the Inventors discovered that the degree of decrease in the impedance between electrodes (i.e., an impedance change rate relative to temperature change of the solid electrolyte member 30) was more prominent between the electrodes with low impedance than between the electrodes with high impedance. Namely, as shown in the graph of Fig. 9, particularly, in the region where the temperature of the solid electrolyte member 30 is not less than 200°C, the impedance decrease rate is largest between electrode F1 and electrode B1 with the shortest distance, and the impedance decrease rate is next largest between electrode F1 and electrode F2. In contrast, the impedance decrease rate is small between electrode F1 and electrode F3, and the impedance decrease rate is smallest between electrode F1 and electrode B3 with the largest distance. Therefore, the impedance difference between electrodes located at different distances from the electrode F1 (i.e., between electrodes with different impedances in the solid electrolyte member 30 with respect to electrode F1) enlarges with increase in the temperature of the solid electrolyte member 30. This permits the current temperature of the solid electrolyte member 30 to be measured based on the difference of impedances between electrodes located at different distances from the electrode F1 (impedances with respect to the electrode F1). The gas concentration calculator 9c applies this phenomenon to determination of the temperature of the solid electrolyte member 3 on the basis of the difference of electromotive forces between each of the reference electrodes 5a and 5b and the sensing electrode 4, from the fact that the impedance between electrodes appears as an electromotive force.

The gas concentration calculator 9c is electrically connected to control terminals (not shown) for controlling the conducting states of the switches 7a and 7b. In order to individually measure the electromotive forces at the respective reference electrodes 5a and 5b, the gas concentration calculator 9c is arranged to be able to feed an on/off signal S₄ to the control terminals of the switches 7a and 7b to control the conducting states of the switches 7a and 7b.

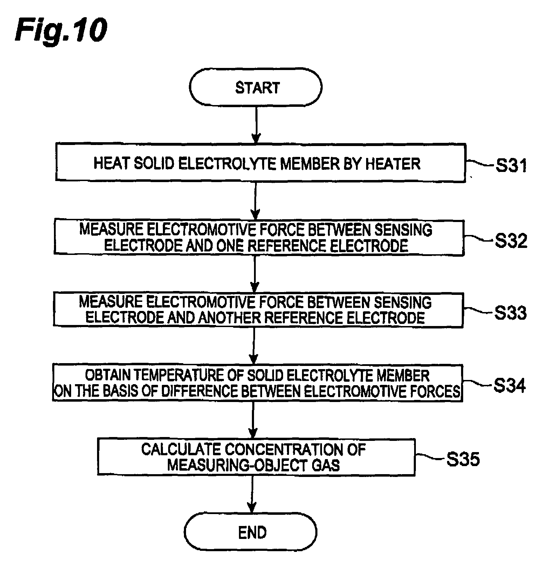

The configuration of gas sensor 1c according to the present embodiment was described above. Subsequently, a gas concentration measurement method according to the present embodiment will be described below together with the operation of gas sensor 1c with reference to Fig. 10. Fig. 10 is a flowchart showing the operation of gas sensor 1c. In the description hereinafter, the method and operation will be described using carbon dioxide as a measuring-object gas and lithium carbonate as a material included in the sensing electrode 4.

First, a power is supplied from heater power supply 10 to heater 6, whereby the heater' 6 generates heat. Then the solid electrolyte member 3 is heated to a high temperature, e.g., 350°C or higher (heating step, S31). This results in changing lithium carbonate in the sensing electrode 4 into an active state as Li₂CO₃ is separated and ionized into 2Li⁺ and CO₃²⁻. 2Li⁺ react with oxygen (O₂) or carbon dioxide (CO₂) entering the sensing electrode 4 from the outside, as indicated by the reaction of (1) above, to form new Li₂CO₃. Li₂CO₃ newly formed reacts with Na ions being conductive ions in the solid electrolyte member 3, as indicated by the reaction of (2) above. In the above reaction, Li₂CO₃ releases electrons in the reaction with the Na ions. The electrons thus released are taken into Na₂CO₃ to induce an electromotive force between the sensing electrode 4 and the reference electrodes 5a and 5b.

Subsequently, the gas concentration calculator 9c closes the switch 7a (to bring it into a conducting state). This results in feeding an electromotive force between sensing electrode 4 and reference electrode 5a to the input terminals 8a and 8b of amplifier 8, and the amplifier 8 amplifies the electromotive force to produce an electromotive force signal S₁ and provides it to the gas concentration calculator 9c. The gas concentration calculator 9c stores this electromotive force signal S₁ as the electromotive force (first electromotive force) at the reference electrode 5a in a memory (electromotive force measuring step, S32).

Subsequently, the gas concentration calculator 9c opens the switch 7a (to bring it into a non-conducting state) and closes the switch 7b (to bring it into a conducting state). This results in feeding an electromotive force between sensing electrode 4 and reference electrode 5b to the input terminals 8a and 8b of amplifier 8, and the amplifier 8 amplifies the electromotive force to produce an electromotive force signal S₁ and provides it to the gas concentration calculator 9c. The gas concentration calculator 9c stores this electromotive force signal S₁ as the electromotive force (second electromotive force) at the reference electrode 5b in the memory (electromotive force measuring step, S33).

The gas concentration calculator 9c obtains the temperature of the solid electrolyte member 3, based on the difference between the first electromotive force and the second electromotive force thus provided (gas concentration calculating step, S34). At this time, the gas concentration calculator 9c is preferably configured to preliminarily store correlation data between the difference of the electromotive forces at the respective reference electrodes 5a and 5b (e.g., electromotive force difference) and the temperature of solid electrolyte member 3 and to apply the difference between the first electromotive force and the second electromotive force to this correlation data, thereby obtaining the temperature of the solid electrolyte member 3.

After obtaining the temperature of the solid electrolyte member 3, the gas concentration calculator 9c obtains the concentration of carbon dioxide, based on a correlation between electromotive force and concentration of carbon dioxide (gas concentration calculating step, S35). Namely, the first and second electromotive force values measured depend mainly upon the concentration of carbon dioxide and the temperature of solid electrolyte member 3, and thus the concentration of carbon dioxide can be obtained once the temperature of the solid electrolyte member 3 is determined. The gas concentration calculator 9c is preferably configured to preliminarily store correlation data between the electromotive forces at the respective reference electrodes 5a and 5b and the carbon dioxide concentration at each temperature and to apply the first electromotive force value and/or the second electromotive force value and the current temperature of the solid electrolyte member 3 to this correlation data, thereby obtaining the concentration of carbon dioxide.

The gas concentration measurement method and gas sensor 1c according to the present embodiment have the following effect. Namely, the gas concentration measurement method and gas sensor 1c according to the present embodiment are arranged to obtain the temperature of the solid electrolyte member 3 on the basis of the difference of the electromotive forces at the respective reference electrodes 5a and 5b, whereby the temperature of the solid electrolyte member 3 can be measured without use of the temperature detecting element. Since the temperature of the solid electrolyte member 3 can be directly measured, the temperature of the solid electrolyte member 3 can be measured with higher accuracy than with use of the temperature detecting element. This permits the concentration of the measuring-object gas such as carbon dioxide to be determined easily and accurately.

In the gas concentration measurement method and gas sensor 1c of the present embodiment, the plurality of reference electrodes 5a and 5b preferably have their respective distances different from each other with respect to the sensing electrode 4. This permits the reference electrodes 5a and 5b to be suitably arranged so that the reference electrodes 5a and 5b have their respective impedances different from each other in the solid electrolyte member 3 with respect to the sensing electrode 4.

The gas sensor 1c is preferably provided with the gas concentration calculator 9c for obtaining the temperature of the solid electrolyte member 3 on the basis of the difference of electromotive forces between the sensing electrode 4 and each of the reference electrodes 5a and 5b and for obtaining the concentration of the measuring-object gas, based on the correlation between the electromotive force and the concentration of the measuring-object gas corresponding to the temperature as in the present embodiment. This permits the temperature of the solid electrolyte member 3 to be accurately measured without use of the temperature detecting element and suitably realizes the gas sensor capable of readily and accurately measuring the concentration of the measuring-object gas. Although the present embodiment is arranged to make the gas concentration calculator 9c automatically calculate the concentration of the measuring-object gas, the gas concentration measurement method according to the present invention may be arranged to make an arithmetic operation means outside the gas sensor 1c calculate the concentration of the measuring-object gas, or to permit a measuring person to perform the calculation by himself or herself

The gas sensor 1c is preferably provided with the amplifying means such as amplifier 8 as in the present embodiment. This permits the first and second electromotive forces to be measured with higher accuracy between each of the reference electrodes 5a and 5b and the sensing electrode 4.

The gas sensor 1c is preferably provided with a plurality of switches 7a and 7b according to the plurality of reference electrodes 5a and 5b as in the present embodiment. This permits the gas concentration calculator 9c to suitably select one of the reference electrodes 5a and 5b in the measurement of electromotive force, which can achieve downsizing of the circuit for measuring the electromotive forces between each of the reference electrodes 5a and 5b and the sensing electrode 4 (e.g., amplifier 8).

### (Modification Example)

The gas sensor 1c may comprise the sensor element 2b shown in Fig. 4 instead of the sensor element 2a. The plurality of reference electrodes 5c, 5d with mutually different impedances in the solid electrolyte member 3 with respect to the sensing electrode 4 can also be suitably realized by arranging the reference electrodes 5c, 5d so as to have their respective contact areas different from each other with respect to the solid electrolyte member 3 as in the present modification example.

### (Fourth Embodiment)

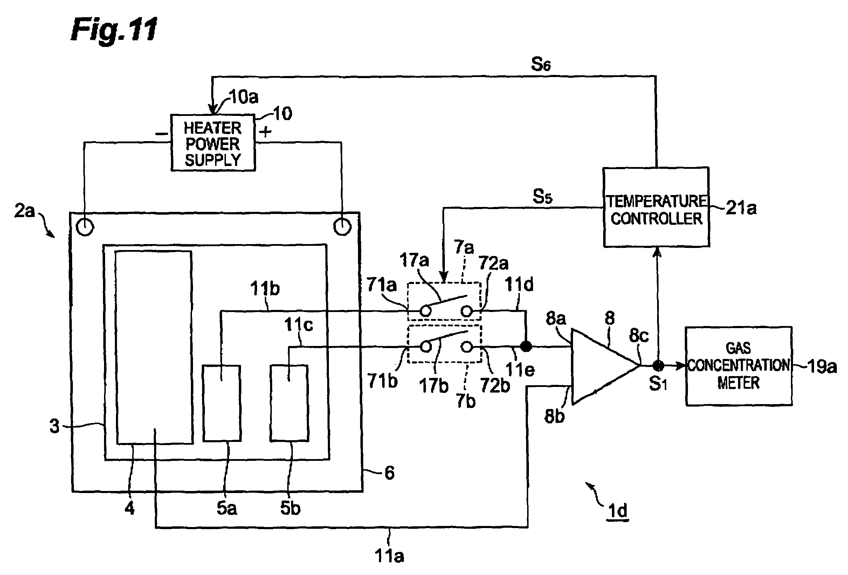

Subsequently, the fourth embodiment of the gas concentration measurement method and gas sensor according to the present invention will be described. Fig. 11 is a schematic diagram showing a configuration of gas sensor 1d according to the present embodiment. The gas sensor 1d is provided with sensor element 2a, switches 7a and 7b, amplifier 8, heater power supply 10, wires 11a-11e, gas concentration meter (gas concentration measuring means) 19a, and temperature controller (temperature controlling means) 21a. Among these, the sensor element 2a, switches 7a and 7b, amplifier 8, heater power supply 10, and wires 11a-11e are constructed in much the same configuration as in the third embodiment, and the description thereof is thus omitted herein.

The temperature controller 21a is a means for obtaining the temperature of the solid electrolyte member 3 on the basis of the difference of electromotive forces between each of the reference electrodes 5a and 5b and the sensing electrode 4 and for controlling the supplied power to the heater 6 so that the temperature nears a predetermined temperature. The procedure of obtaining the temperature of the solid electrolyte member 3 in the temperature controller 21a is similar to that in the gas concentration calculator 9c in the third embodiment. The temperature controller 21a can be realized, for example, in such a manner that an arithmetic operation device such as a CPU storing a map of characteristics prepared in advance executes an arithmetic operation according to a predetermined program. The map of characteristic values and the program may be used as stored in an external storage device, for example, such as a hard disk or a memory The temperature controller 21a is electrically connected to the output terminal 8c of the amplifier 8 and receives the electromotive force signal S₁ from the output terminal 8c. The temperature controller 21a is also electrically connected to control terminals (not shown) for controlling the conducting states of the switches 7a and 7b. In order to individually measure the electromotive forces at the respective reference electrodes 5a and 5b, the temperature controller 21a is able to feed an on/off signal S₅ to the control terminals of the switches 7a and 7b and thereby to control the conducting states of the switches 7a and 7b. The temperature controller 21a is electrically connected to a control terminal 10a for controlling the output of the heater power supply 10, and provides a power control signal S₆ for contcolling the supplied power to the heater 6, to the heater power supply 10.

The gas concentration meter 19a is a means for measuring an electromotive force between at least one of the reference electrodes 5a and 5b and the sensing electrode 4 (third electromotive force) and for determining the concentration of carbon dioxide, based on a correlation between the electromotive force and the carbon dioxide concentration corresponding to the predetermined temperature set by the temperature controller 21a. The gas concentration meter 19a can be realized, for example, in such a manner that an arithmetic operation device such as a CPU storing a map of characteristics prepared in advance executes an arithmetic operation according to a predetermined program. The map of characteristic values and the program may be used as stored in an external storage device, for example, such as a hard disk or a memory. The gas concentration meter 19a is electrically connected to the output terminal 8c of the amplifier 8 and receives the electromotive force signal S₁ from the output terminal 8c. The gas concentration meter 19a preliminarily stores correlation data between the electromotive force at the reference electrode 5a and/or 5b and the carbon dioxide concentration at the predetermined temperature. The gas concentration meter 19a applies the electromotive force signal S₁ to this correlation data, thereby obtaining the carbon dioxide concentration.

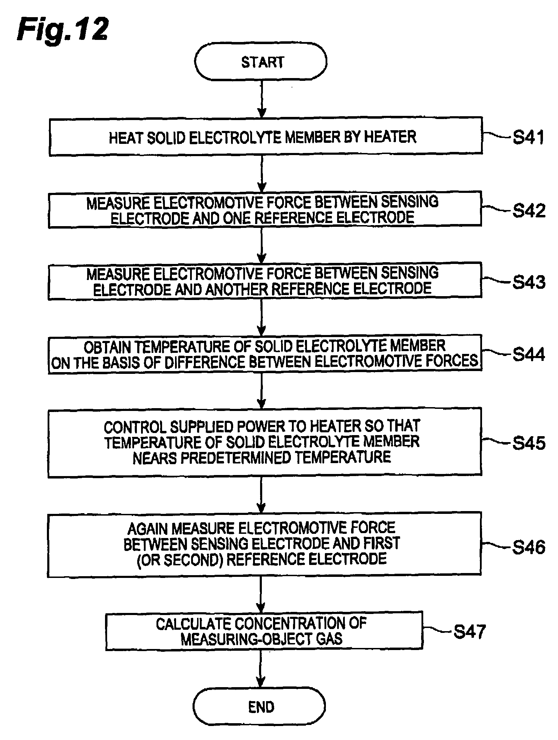

Subsequently, a gas concentration measurement method according to the present embodiment will be described together with the operation of gas sensor 1d with reference to Fig. 12. Fig. 12 is a flowchart showing the operation of gas sensor 1d.

First, the power is supplied from the heater power supply 10 to the heater 6, whereby the heater 6 generates heat. Then the solid electrolyte member 3 is heated to a high temperature, e.g., 350°C or higher (heating step, S41). This results in inducing an electromotive force according to the concentration of carbon dioxide between the sensing electrode 4 and the reference electrodes 5a and 5b.

Subsequently, the temperature controller 21a closes the switch 7a (to bring it into a conducting state). This results in feeding the electromotive force between sensing electrode 4 and reference electrode 5a to the input terminals 8a and 8b of the amplifier 8, and the amplifier 8 amplifies it to produce the electromotive force signal S₁, and provides the signal to the temperature controller 21 a. The temperature controller 21a stores this electromotive force signal S₁ as the electromotive force at the reference electrode 5a (first electromotive force) in a memory (electromotive force measuring step, S42).

Subsequently, the temperature controller 21a opens the switch 7a (to bring it into a non-conducting state) and closes the switch 7b (to bring it into a conducting state). This results in feeding the electromotive force between sensing electrode 4 and reference electrode 5b to the input terminals 8a and 8b of the amplifier 8, and the amplifier 8 amplifies it to produce the electromotive force signal S₁, and provides the signal to the temperature controller 21a. The temperature controller 21a stores this electromotive force signal S₁ as the electromotive force at the reference electrode 5b (second electromotive force) in the memory (electromotive force measuring step, S43).

The temperature controller 21a determines the temperature of the solid electrolyte member 3, based on the difference between the first electromotive force and the second electromotive force thus provided (temperature control step, S44). At this time, the temperature controller 21a is preferably configured to preliminarily store correlation data between the difference of electromotive forces at the respective reference electrodes 5a and 5b (e.g., electromotive force difference) and the temperature of solid electrolyte member 3 and to apply the difference between the first electromotive force and the second electromotive force to this correlation data, thereby obtaining the temperature of the solid electrolyte member 3.

After obtaining the temperature of the solid electrolyte member 3, the temperature controller 21a controls the supplied power to the heater 6 so that the solid electrolyte member 3 nears the predetermined temperature (temperature control step, S45). Namely, the temperature controller 21a feeds the power control signal S₆ to the heater power supply 10, based on the difference between the current temperature of the solid electrolyte member 3 and the predetermined temperature, to control the output (supplied power) from the heater power supply 10. The temperature controller 21a is preferably configured to repeat the above operation until the temperature of the solid electrolyte member 3 becomes stabilized. In this manner, the temperature of the solid electrolyte member 3 is controlled to the predetermined temperature or to a temperature close to the predetermined temperature.

After that, the gas concentration meter 19a again measures the electromotive force between the reference electrode 5a and/or 5b and the sensing electrode 4 (gas concentration measuring step, S46). Namely, after the temperature of the solid electrolyte member 3 becomes stabilized at the predetermined temperature, the gas concentration meter 19a receives the electromotive force signal S₁ at both or at least one of the reference electrodes 5a and 5b from the amplifier 8. Then the gas concentration meter 19a obtains the carbon dioxide concentration, based on the correlation data between the electromotive force signal S₁ at the reference electrode 5a and/or 5b and the carbon dioxide concentration (gas concentration measuring step, S47).

Since the gas concentration measurement method of the present embodiment is arranged to obtain the temperature of the solid electrolyte member 3 on the basis of the difference of the electromotive forces at the respective reference electrodes 5a and 5b in the same manner as the gas concentration measurement method of the third embodiment, the temperature of the solid electrolyte member 3 can be measured without use of the temperature detecting element. Since the temperature of the solid electrolyte member 3 can be directly measured, the temperature of the solid electrolyte member 3 can be measured with higher accuracy than with use of the temperature detecting element. Accordingly, the temperature of the solid electrolyte member 3 can be controlled easily and accurately.

The gas sensor 1d is preferably provided with the temperature controller 21 a for obtaining the temperature of the solid electrolyte member 3 on the basis of the difference of electromotive forces between each of the reference electrodes 5a and 5b and the sensing electrode 4 and for controlling the supplied power to the heater 6 so that the temperature nears the predetermined temperature as in the present embodiment. This suitably realizes the gas sensor 1d capable of accurately measuring the temperature of the solid electrolyte member 3 without use of the temperature detecting element and easily and accurately controlling the temperature. The present embodiment is arranged to make the temperature controller 21a automatically perform the temperature control of the solid electrolyte member 3, but the gas concentration measurement method according to the present invention may also be configured to make a controller outside the gas sensor 1d perform the temperature control of the solid electrolyte member 3, or to permit a measuring person to perform the control by himself or herself.

### (Fifth Embodiment)

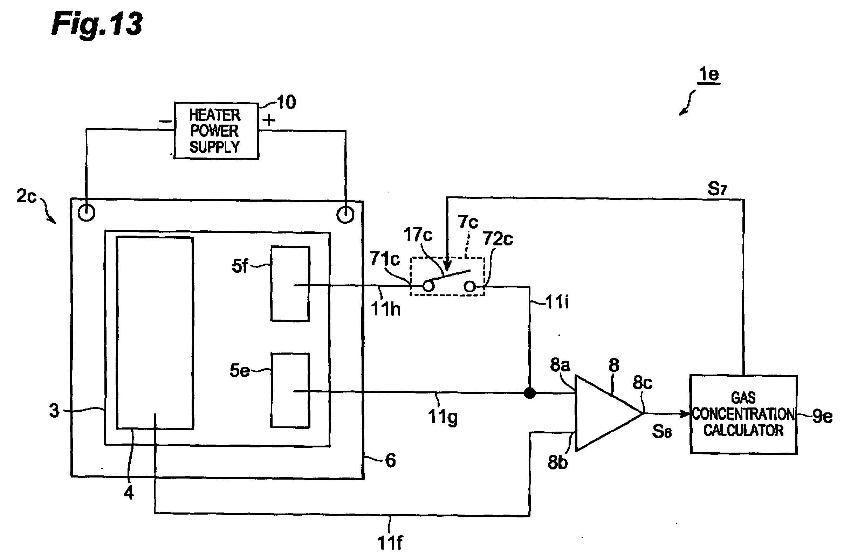

Subsequently, the fifth embodiment of the gas concentration measurement method and gas sensor according to the present invention will be described. Fig. 13 is a schematic diagram showing a configuration of gas sensor 1e according to the present embodiment. The gas sensor 1e is provided with sensor element 2c, switch 7c, amplifier 8, gas concentration calculator (gas concentration calculating means) 9e, heater power supply 10, and wires 11f-11i. Among these components, the sensor element 2c, switch 7c, amplifier 8, and wires 11f-11i are constructed in much the same configuration as in the second embodiment, and the description thereof is omitted herein. In addition, the heater power supply 10 is different from the heat power supply 10 of the second embodiment in the configuration in which the temperature controller 19 is not connected to the heat power supply 10 of the present embodiment. The switch 7c has a control terminal (not shown) for controlling the conducting states thereof.

The gas concentration calculator 9e is a means for obtaining the temperature of the solid electrolyte member 3 on the basis of the difference between the electromotive force between reference electrode 5e and sensing electrode 4 (first electromotive force) and the electromotive force between the reference electrodes 5e and 5f short-circuited by the switch 7c, and the sensing electrode 4 (second electromotive force) and for obtaining the concentration of the measuring-object gas, based on the correlation between the electromotive force and the concentration of the measuring-object gas corresponding to the temperature.

The gas concentration calculator 9e is electrically connected to a control terminal (not shown) for controlling the conducting state of the switch 7c. In order to individually measure the electromotive force at the single reference electrode 5e and the electromotive force in the mutually short-circuited state of the reference electrodes 5e and 5f (in the enlarged reference electrode state), the gas concentration calculator 9e is able to feed an on/off signal S₇ to the control terminal of the switch 7c to control the conducting state of the switch 7c.

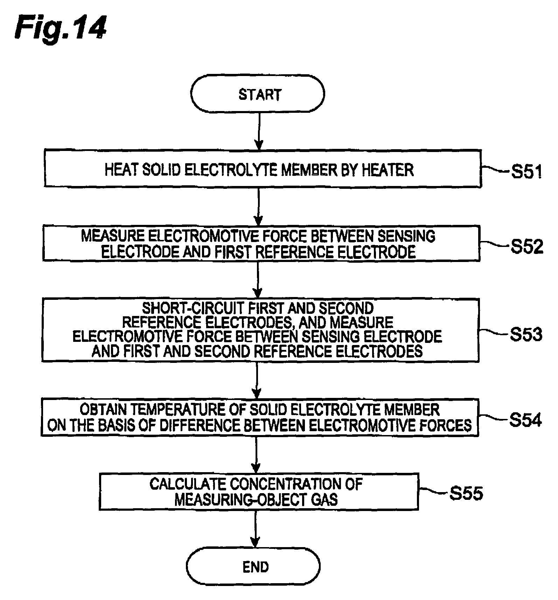

Subsequently, a gas concentration measurement method according to the present embodiment will be described together with the operation of gas sensor 1e with reference to Fig. 14. Fig. 14 is a flowchart showing the operation of gas sensor 1e.

First, the power is supplied from the heater power supply 10 to the heater 6, whereby the heater 6 generates heat. Then the solid electrolyte member 3 is heated to a high temperature, e.g., 350°C or higher (heating step, S51). This results in inducing an electromotive force according to a carbon dioxide concentration between the sensing electrode 4 and the reference electrodes 5e and 5f.

The gas concentration calculator 9e first opens the switch 7c (to bring it into a non-conducting state). This results in feeding the electromotive force between sensing electrode 4 and reference electrode 5e to the input terminals 8a and 8b of the amplifier 8. The amplifier 8 amplifies the electromotive force to produce the electromotive force signal S₈ and provides the signal to the gas concentration calculator 9e. The gas concentration calculator 9e stores this electromotive force signal S₈ as the electromotive force at the reference electrode 5e (first electromotive force) in the memory (electromotive force measuring step, S52).

Subsequently, the gas concentration calculator 9e closes the switch 7c (to bring it into a conducting state). This results in short-circuiting the reference electrodes 5e and 5f through the switch 7c. Namely, the contact area between the reference electrode and the solid electrolyte member 3 is enlarged, so that the impedance is decreased in the solid electrolyte member 3 between the reference electrode and the sensing electrode 4. Then the electromotive force induced between the short-circuited reference electrodes 5e and 5f and the sensing electrode 4 is fed to the input terminals 8a and 8b of the amplifier 8, and the amplifier 8 amplifies the electromotive force to produce the electromotive force signal S₈ and provides the signal to the gas concentration calculator 9e. The gas concentration calculator 9e stores this electromotive force signal S₈ as the electromotive force at the short-circuited reference electrodes 5e and 5f (second electromotive force) in the memory (electromotive force measuring step, S53).

The gas concentration calculator 9e obtains the temperature of the solid electrolyte member 3, based on the difference between the first electromotive force and the second electromotive force thus provided (gas concentration calculating step, S54). At this time, the gas concentration calculator 9e is preferably arranged to preliminarily store correlation data between the difference between the electromotive force at the single reference electrode 5e and the electromotive force in the short-circuited state of the reference electrodes 5e and 5f (e.g., electromotive force difference), and the temperature of the solid electrolyte member 3, and to apply the difference between the first electromotive force and the second electromotive force to this correlation data, thereby obtaining the temperature of the solid electrolyte member 3.

After obtaining the temperature of the solid electrolyte member 3, the gas concentration calculator 9e obtains the carbon dioxide concentration, based on the correlation between the electromotive force and the carbon dioxide concentration (gas concentration calculating step, S55). Namely, since the first and second electromotive force values measured depend upon the concentration of carbon dioxide and the temperature of solid electrolyte member 3, the carbon dioxide concentration can be determined once the temperature of the solid electrolyte member 3 is obtained. The gas concentration calculator 9e is preferably configured to preliminarily store correlation data of carbon dioxide concentration with the electromotive force at the single reference electrode 5e and/or the electromotive force in the short-circuited state of the reference electrodes 5e and 5f at each temperature, and to apply the first electromotive force value and/or the second electromotive force value and the temperature of the solid electrolyte member 3 to this correlation data, thereby obtaining the carbon dioxide concentration.

The gas concentration measurement method and gas sensor 1e according to the present embodiment have the following effect. Namely, the gas concentration measurement method and gas sensor 1e according to the present embodiment are arranged to measure the electromotive force between one of two reference electrodes 5e and 5f and the sensing electrode 4 (first electromotive force) and to further measure the electromotive force between the sensing electrode 4 and the reference electrodes 5e and 5f in the mutually short-circuited state of the two reference electrodes 5e and 5f (i.e., in the enlarged contact area state of the reference electrode with the solid electrolyte member 3). By changing the contact area between the reference electrode and the solid electrolyte member 3 in this manner, it becomes feasible to suitably change the impedance between the reference electrode and sensing electrode 4. Therefore, the temperature of the solid electrolyte member 3 can be determined based on the difference between the first electromotive force and the second electromotive force. Since the gas concentration measurement method and gas sensor 1e of the present embodiment are arranged to obtain the temperature of the solid electrolyte member 3 on the basis of the difference between the first electromotive force and the second electromotive force, the temperature of the solid electrolyte member 3 can be measured without use of the temperature detecting element. Since the temperature of the solid electrolyte member 3 can be directly measured, the temperature of the solid electrolyte member 3 can be measured with higher accuracy than with use of the temperature detecting element. This permits the concentration of the measuring-object gas to be obtained easily and accurately.

(Sixth Embodiment)

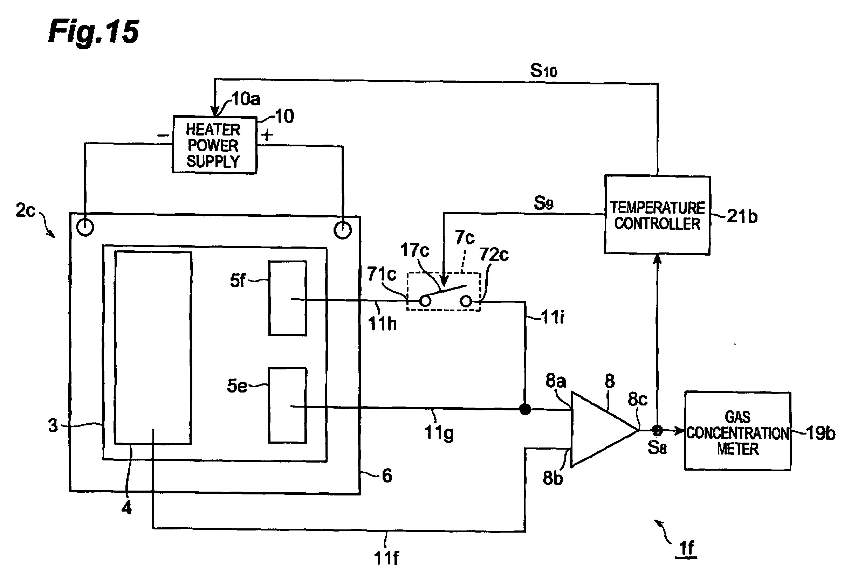

Subsequently, the sixth embodiment of the gas concentration measurement method and gas sensor according to the present invention will be described. Fig. 15 is a schematic diagram showing a configuration of gas sensor 1f according to the present embodiment. The gas sensor 1f is provided with sensor element 2c, switch 7c, amplifier 8, heater power supply 10, wires 11f-11i, gas concentration meter (gas concentration measuring means) 19b, and temperature controller 21b. Among these, the sensor element 2c, switch 7c, amplifier 8, heater power supply 10, and wires 11f-11i are constructed in much the same configuration as in the fifth embodiment, and the description thereof is omitted herein.

The temperature controller 21b is a means for obtaining the temperature of the solid electrolyte member 3 on the basis of the difference between the electromotive force between reference electrode 5e and the sensing electrode 4 (first electromotive force) and the electromotive force between the sensing electrode 4 and the reference electrodes 5e and 5f in the short-circuited state of reference electrodes 5e and 5f (second electromotive force), and for controlling the- supplied power to the heater 6 so that the temperature nears a predetermined temperature. The procedure of obtaining the temperature of the solid electrolyte member 3 in the temperature controller 21b is similar to that in the gas concentration calculator 9e in the fifth embodiment. The temperature controller 21b is electrically connected to the output terminal 8c of the amplifier 8 and receives the electromotive force signal S₈ from the output terminal 8c. The temperature controller 21b is electrically connected to the control terminal (not shown) for controlling the conducting state of the switch 7c. In order to individually measure the electromotive force at the single reference electrode 5e and the electromotive force in the short-circuited state of reference electrodes 5e and 5f, the temperature controller 21b is able to feed the on/off signal S₉ to the control terminal of the switch 7c to control the conducting state of the switch 7c. The temperature controller 21b is electrically connected to the control terminal 10a for controlling the output of heater power supply 10, and provides a power control signal S₁₀ for controlling the supplied power to the heater 6, to the heater power supply 10.

The gas concentration meter 19b is a means for measuring the electromotive force between reference electrode 5e and sensing electrode 4 or the electromotive force between the reference electrodes 5e and 5f in the short-circuited state of the reference electrodes 5e and 5f, and the sensing electrode 4 (third electromotive force), and for obtaining the carbon dioxide concentration, based on the correlation between the electromotive force and the carbon dioxide concentration corresponding to the predetermined temperature set by the temperature controller 21b. The gas concentration meter 19b is electrically connected to the output terminal 8c of amplifier 8 and receives the electromotive force signal S₈ from the output terminal 8c. The gas concentration meter 19b preliminarily stores correlation data between the carbon dioxide concentration and the electromotive force at the single reference electrode 5e and/or the electromotive force in the short-circuited state of the reference electrodes 5e and 5f at the predetermined temperature. The gas concentration meter 19b applies the electromotive force signal S₈ to this correlation data, thereby obtaining the carbon dioxide concentration.

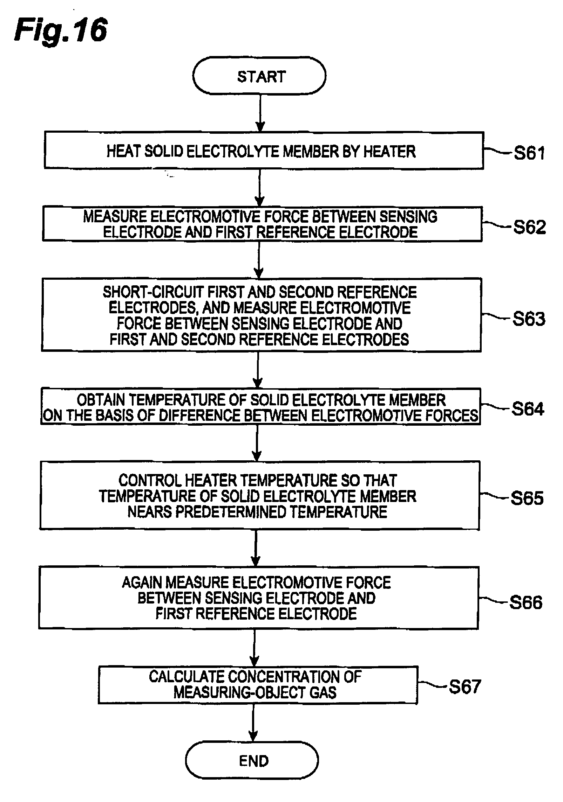

Subsequently, a gas concentration measurement method according to the present embodiment will be described together with the operation of gas sensor 1f with reference to Fig. 16. Fig. 16 is a flowchart showing the operation of gas sensor 1f.

First, the power is supplied from the heater power supply 10 to the heater 6, whereby the heater 6 generates heat. Then the solid electrolyte member 3 is heated to a high temperature, e.g., 350°C or higher (heating step, S61). This results in inducing an electromotive force according to a carbon dioxide concentration between the sensing electrode 4 and the reference electrodes 5e and 5f

The temperature controller 21b first opens the switch 7c (to bring it into a non-conducting state). This results in feeding the electromotive force between the sensing electrode 4 and reference electrode 5e to the input terminals 8a and 8b of the amplifier 8, and the amplifier 8 amplifies the electromotive force to produce the electromotive force signal S₈, and provides the signal to the temperature controller 21b. The temperature controller 21b stores this electromotive force signal S₈ as the electromotive force at the reference electrode 5e (first electromotive force) in the memory (electromotive force measuring step, S62).

Subsequently, the temperature controller 21b closes the switch 7c (to bring it into a conducting state). This results in short-circuiting the reference electrodes 5e and 5f through the switch 7c. Namely, the contact area is enlarged between the reference electrode and the solid electrolyte member 3, so that the impedance is decreased in the solid electrolyte member 3 between the reference electrode and the sensing electrode 4. Then the electromotive force between the short-circuited reference electrodes 5e and 5f and the sensing electrode 4 is fed to the input terminals 8a and 8b of the amplifier 8, and the amplifier 8 amplifies the electromotive force to produce the electromotive force signal S₈, and provides the signal to the temperature controller 21b. The temperature controller 21b stores this electromotive force signal S₈ as the electromotive force at the short-circuited reference electrodes 5e and 5f (second electromotive force) in the memory (electromotive force measuring step, S63).

The temperature controller 21b obtains the temperature of solid electrolyte member 3, based on the difference between the first electromotive force and the second electromotive force thus provided (temperature control step, S64). At this time, the temperature controller 21b is preferably configured to preliminarily store correlation data between the difference between the electromotive force at the single reference electrode 5e and the electromotive force in the short-circuited state of the reference electrodes 5e and 5f (e.g., electromotive force difference), and the temperature of the solid electrolyte member 3, and to apply the difference between the first electromotive force and the second electromotive force to this correlation data, thereby obtaining the temperature of the solid electrolyte member 3.

After obtaining the temperature of the solid electrolyte member 3, the temperature controller 21b controls the supplied power to heater 6 so that the solid electrolyte member 3 nears the predetermined temperature (temperature control step, S65). Namely, the temperature controller 21b feeds the power control signal S₁₀ to the heater power supply 10, based on the difference between the current temperature of the solid electrolyte member 3 and the predetermined temperature, to control the output (supplied power) from the heater power supply 10. In this manner, the temperature of the solid electrolyte member 3 is controlled to the predetermined temperature or to a temperature close to the predetermined temperature.

Thereafter, the gas concentration meter 19b again measures the electromotive force between reference electrode 5e and sensing electrode 4 or the electromotive force between the reference electrodes 5e and 5f in the short-circuited state of the reference electrodes 5e and 5f, and the sensing electrode 4 (gas concentration measuring step, S66). Namely, after the temperature of the solid electrolyte member 3 becomes stabilized at the predetermined temperature, the gas concentration meter 19b receives the electromotive force signal S₈ in the conducting state or in the non-conducting state of the switch 7c from the amplifier 8. Then the gas concentration meter 19b determines the carbon dioxide concentration, based on the correlation data between the electromotive force signal S₈ and the carbon dioxide concentration (gas concentration measuring step, S67).

Since the gas concentration measurement method of the present embodiment is arranged to obtain the temperature of the solid electrolyte member 3 on the basis of the difference between the first electromotive force and the second electromotive force in the same manner as the gas concentration measurement method of the fifth embodiment, the temperature of the solid electrolyte member 3 can be measured without use of the temperature detecting element. Since the temperature of the solid electrolyte member 3 can be directly measured, the temperature of the solid electrolyte member 3 can be measured with higher accuracy than with use of the temperature detecting element. This permits the temperature of the solid electrolyte member 3 to be controlled easily and accurately

The gas concentration measurement methods and gas sensors according to the present invention are not limited to the above embodiments, but can be modified in various ways. For example, each of the above embodiments is arranged to heat the solid electrolyte member to the high temperature with use of the heater, but the heater is not always essential if the solid electrolyte member can have ion conductivity even at ordinary temperature. Even in such cases, the temperature of the solid electrolyte member varies with ambient temperature, and thus the gas concentration can be accurately measured by obtaining the temperature of the solid electrolyte member by the gas concentration measurement method according to the present invention.

Each of the above embodiments is arranged to measure carbon dioxide as a measuring-object gas. The measuring-object gas in the present invention can be any gas whose concentration can be measured based on the electromotive force in the solid electrolyte member, and the present invention can also be applied, for example, to oxygen sensors and others.

In each of the above embodiments, the gas sensor is provided with the solid electrolyte member of flat plate shape. The solid electrolyte member is not limited to the flat plate shape, but can be formed in other various shapes.

In each of the above embodiments, the gas sensor is provided with two reference electrodes. The number of reference electrodes does not have to be limited to two, but may be three or more. In this case, the temperature of the solid electrolyte member can be obtained based on the difference of electromotive forces at three or more, respective reference electrodes, and thus the temperature can be obtained with better accuracy

In each of the above embodiments, the sensing electrode and the plurality of reference electrodes are provided each on the same surface of the solid electrolyte member, but the sensing electrode and the plurality of reference electrodes may also be provided on respective surfaces different from each other. The plurality of reference electrodes may also be provided on respective surfaces different from each other.

As described above, the present invention successfully provides the gas concentration measurement method and gas sensor capable of changing the impedance between the reference electrode and the sensing electrode.

In addition, the present invention successfully provides the gas concentration measurement method and gas sensor capable of accurately measuring the temperature of the solid electrolyte member, without need for use of the temperature detecting element.

## Claims

1. A gas sensor comprising:
a solid electrolyte member,
a sensing electrode provided on the solid electrolyte member and comprising at least one of a metal carbonate and a metal hydrogen carbonate; and
first and second reference electrodes arranged on the solid electrolyte member.

2. The gas sensor according to Claim 1, further comprising switch means for short-circuiting the first reference electrode and the second reference electrode with each other.

3. The gas sensor according to Claim 1 or 2, further comprising amplifying means for amplifying a voltage between two input terminals,
wherein one of said two input terminals of the amplifying means is electrically connected to the first reference electrode or the second reference electrode, and
wherein the other of said two input terminals of the amplifying means is electrically connected to the sensing electrode.

4. The gas sensor according to any one of Claims 1 to 3, further comprising gas concentration measuring means for measuring a first electromotive force between one of the first and second reference electrodes and the sensing electrode, or a second electromotive force between the first and second reference electrodes in a mutually short-circuited state of the first and second reference electrodes, and the sensing electrode, and for obtaining a concentration of a measuring-object gas, based on a correlation between the first or second electromotive force and the concentration of the measuring-object gas.

5. The gas sensor according to any one of Claims 1 to 3, further comprising gas concentration calculating means for measuring a first electromotive force between one of the first and second reference electrodes and the sensing electrode, and a second electromotive force between the first and second reference electrodes in a mutually short-circuited state of the first and second reference electrodes, and the sensing electrode, for obtaining a temperature of the solid electrolyte member on the basis of a difference between the first electromotive force and the second electromotive force, and for obtaining a concentration of a measuring-object gas, based on a correlation between the first or second electromotive force and the concentration of the measuring-object gas, corresponding to the temperature.

6. The gas sensor according to any one of Claims 1 to 5, further comprising:
a heater for heating the solid electrolyte member, and
temperature controlling means for controlling a supplied power to the heater so that a temperature of the solid electrolyte member nears a preset temperature.

7. The gas sensor according to any one of Claims 1 to 4, further comprising:
a heater for heating the solid electrolyte member; and
temperature controlling means for measuring a first electromotive force between one of the first and second reference electrodes and the sensing electrode, and a second electromotive force between the first and second reference electrodes in a mutually short-circuited state of the first and second reference electrodes, and the sensing electrode, for obtaining a temperature of the solid electrolyte member on the basis of a difference between the first electromotive force and the second electromotive force, and for controlling a supplied power to the heater so that the temperature nears a predetermined temperature.

8. A gas sensor comprising:
a solid electrolyte member;
a sensing electrode provided on the solid electrolyte member and comprising at least one of a metal carbonate and a metal hydrogen carbonate; and
a plurality of reference electrodes arranged on the solid electrolyte member so as to have mutually different impedances in the solid electrolyte member with respect to the sensing electrode.

9. The gas sensor according to Claim 8, wherein the plurality of reference electrodes have their respective distances different from each other with respect to the sensing electrode.

10. The gas sensor according to Claim 8, wherein the plurality of reference electrodes have their respective contact areas different from each other with respect to the solid electrolyte member.

11. The gas sensor according to any one of Claims 8 to 10, comprising a plurality of switch means for switching a conducting state between two terminals,
wherein one-side terminals of the respective switch means are electrically connected to the respective reference electrodes in one to one relation, and other-side terminals of the respective switch means are short-circuited with each other.

12. The gas sensor according to Claim 11, further comprising amplifying means for amplifying a voltage between two input terminals,
wherein one of said two input terminals of the amplifying means is electrically connected to said other-side terminals of the respective switch means, and
wherein the other of said two input terminals of the amplifying means is electrically connected to the sensing electrode.

13. The gas sensor according to any one of Claims 8 to 12, further comprising a heater for heating the solid electrolyte member.

14. The gas sensor according to Claim 13, further comprising temperature controlling means for obtaining a temperature of the solid electrolyte member on the basis of a difference of electromotive forces between each of the reference electrodes and the sensing electrode and for controlling a supplied power to the heater so that said temperature nears a predetermined temperature.

15. The gas sensor according to any one of Claims 8 to 13, further comprising gas concentration calculating means for obtaining a temperature of the solid electrolyte member on the basis of a difference of electromotive forces between each of the reference electrodes and the sensing electrode and for obtaining a concentration of a measuring-object gas, based on a correlation between the electromotive force at at least one said reference electrode and the concentration of the measuring-object gas, corresponding to the temperature.

16. The gas sensor according to any one of Claims 8 to 13, further comprising gas concentration measuring means for measuring a concentration of a measuring-object gas, based on a correlation between an electromotive force between at least one reference electrode among the plurality of reference electrodes and sensing electrode, and the concentration of the measuring-object gas.

17. A gas concentration measurement method by use of the gas sensor defined in any one of claims 8 to 13 and 16, comprising:
a selecting step of selecting a reference electrode from the plurality of reference electrodes;
an electromotive force measuring step of measuring an electromotive force between the reference electrode selected in the selecting step, and the sensing electrode; and
a gas concentration calculating step of obtaining a concentration of a measuring-object gas, based on a correlation between the electromotive force measured in the electromotive force measuring step and the concentration of the measuring-object gas.

18. A gas concentration measurement method by use of the gas sensor defined in any one of claims I to 4 and 6, comprising:
a selecting step of selecting which electromotive force should be measured between a first electromotive force between one of the first and second reference electrodes and the sensing electrode and a second electromotive force between the first and second reference electrodes in a mutually short-circuited state of the first and second reference electrodes, and the sensing electrode;
an electromotive force measuring step of measuring the first or second electromotive force selected in the selecting step; and
a gas concentration calculating step of obtaining a concentration of a measuring-object gas, based on a correlation between the first or second electromotive force measured in the electromotive force measuring step and the concentration of the measuring-object gas.

19. The gas concentration measurement method according to Claim 17 or 18, further comprising:
a temperature selecting step of selecting a preset temperature of the solid electrolyte member; and
a heating step of heating the solid electrolyte member so that a temperature of the solid electrolyte member nears the preset temperature, prior to the electromotive force measuring step.

20. A gas concentration measurement method by use of the gas sensor defined in any one of claims 8 to 15, comprising:
an electromotive force measuring step of measuring electromotive forces between the plurality of reference electrodes and the sensing electrode; and
a gas concentration calculating step of obtaining a temperature of the solid electrolyte member on the basis of a difference between the electromotive forces, and obtaining a concentration of a measuring-object gas, based on a correlation between an electromotive force at at least one reference electrode among the plurality of reference electrodes and the concentration of the measuring-object gas, corresponding to said temperature.

21. A gas concentration measurement method by use of the gas sensor defined in any one of claims 8 to 15, comprising:
an electromotive force measuring step of measuring electromotive forces between the plurality of reference electrodes and the sensing electrode;
a temperature control step of obtaining a temperature of the solid electrolyte member on the basis of a difference between the electromotive forces, and controlling an amount of heat to the solid electrolyte member so that said temperature nears a predetermined temperature; and
a gas concentration measuring step of obtaining a concentration of a measuring-object gas, based on a correlation between an electromotive force between at least one reference electrode out of the plurality of reference electrodes and the sensing electrode, and the concentration of the measuring-object gas, corresponding to the predetermined temperature.

22. A gas concentration measurement method by use of the gas sensor defined in any one of claims 1 to 3, 5, and 6, comprising:
an electromotive force measuring step of measuring a first electromotive force between one of the first and second reference electrodes and the sensing electrode, and measuring a second electromotive force between the first and second reference electrodes and the sensing electrode in a short-circuited state of the first and second reference electrodes; and
a gas concentration calculating step of obtaining a temperature of the solid electrolyte member on the basis of a difference between the first electromotive force and the second electromotive force and obtaining a concentration of a measuring-object gas, based on a correlation between the first or second electromotive force and the concentration of the measuring-object gas corresponding to the temperature.

23. A gas concentration measurement method by use of the gas sensor defined in any one of claims 1 to 4, and 7, comprising:
an electromotive force measuring step of measuring a first electromotive force between one of the first and second reference electrodes and the sensing electrode, and measuring a second electromotive force between the first and second reference electrodes and the sensing electrode in a short-circuited state of the first and second reference electrodes;
a temperature control step of obtaining a temperature of the solid electrolyte member on the basis of a difference between the first electromotive force and the second electromotive force and controlling an amount of heat to the solid electrolyte member so that said temperature nears a predetermined temperature; and
a gas concentration measuring step of measuring a third electromotive force between at least one of the first and second reference electrodes and the sensing electrode, and obtaining a concentration of a measuring-object gas, based on a correlation between the third electromotive force and the concentration of the measuring-object gas corresponding to the predetermined temperature.

24. The gas concentration measurement method according to any one of Claims 20 to 23, further comprising a step of heating the solid electrolyte member, prior to the electromotive force measuring step.
